# EUROPEAN PATENT SPECIFICATION

(11) **EP 3 698 846 B1**
(45) Date of publication and mention of the grant of the patent: **16.10.2024**
(21) Application number: 17930004.1
(22) Date of filing: 27.10.2017
(51) Int. Cl.: A61N 1/39, A61B 5/24, A61N 1/04, A61B 5/024

(54) **DEFIBRILLATOR, STATE DETECTION MANAGEMENT METHOD, STATE DETECTION MANAGEMENT SYSTEM AND DEVICE**
DEFIBRILLATOR, ZUSTANDSERFASSUNGSVERWALTUNGSVERFAHREN, ZUSTANDSERFASSUNGSVERWALTUNGSSYSTEM UND VORRICHTUNG
DÉFIBRILLATEUR, PROCÉDÉ DE GESTION DE DÉTECTION D'ÉTAT, SYSTÈME ET DISPOSITIF DE GESTION DE DÉTECTION D'ÉTAT

(43) Date of publication of application: 26.08.2020
(73) Proprietor: Shenzhen Mindray Bio-Medical Electronics Co., Ltd., Shenzhen, Guangdong 518057 (CN); Shenzhen Mindray Scientific Co., Ltd., Shenzhen, Guangdong 518000 (CN)
(72) Inventor: WANG, Qi, Shenzhen Guangdong 518057 (CN); CHEN, Dabing, Shenzhen Guangdong 518057 (CN); LI, Zhiwei, Shenzhen Guangdong 518057 (CN); LI, Liya, Shenzhen Guangdong 518057 (CN); CEN, Jian, Shenzhen Guangdong 518057 (CN)
(74) Representative: KIPA AB
(86) International application number: PCT/CN2017/108043
(87) International publication number: WO 2019/080098

## Description

### Technical Field

The present invention is set out in the appended claims and generally relates to the field of defibrillators.

### Background Art

Defibrillators are high-risk emergency devices, which are critical, although they are not often used. The defibrillators are mainly used for performing defibrillation treatment on dangerous diseases such as ventricular fibrillation and atrial fibrillation.

In order to ensure that the defibrillators are in a normal state when in use, the defibrillators are generally detected regularly. For example, some of the defibrillators have a status detection function. The status detection function of the defibrillator is a function to find a defibrillator failure in advance and notify the maintenance personnel to perform maintenance processing, and finally reach the state that the defibrillator is ready for use. When classified according to the functional modules, the existing status detection of the defibrillators mainly includes: a battery status detection item, a charging function detection item, a discharging function detection item, an electrocardiogram front-end sampling function detection item, and other items of detection. The problems can be found in time through the status detection, which need to be handled and repaired in a timely manner by the device maintenance personnel, otherwise they will cause the machine to malfunction and cannot be used for rescue during clinical use.

Automatic external defibrillators (AEDs) are defibrillators that are expected to be used in public places (e.g., crowded places such as airports and stations). Unlike the conventional defibrillators in hospitals, AEDs are generally equipped with primary batteries and defibrillation electrode pads. Since the use occasion is a public place, the maintenance and status detection status of the AED is often not available in time, which may lead to the risk that the device cannot be used in actual use.

Moreover, the frequent status detection of the AED will consume the power from the primary battery, thereby reducing the standby life of the battery, which may make the clinical risk of battery power shortage when the AED actually needs to be used.

US 2011/0213433 A1 discloses a battery powered systems with long standby times, such as automatic external defibrillators (AEDs). The system indicates their operational status to a user by blinking lights or sounding speakers or buzzers. These active status indication activities consume power thereby reducing the battery life of the system. To conserve power and to be more effective in seeking attention from a human operator, the status alerts for the AED produced by an active status indicator (ASI) system can be more meaningful to humans or more unique relative to status alerts provided by conventional devices.

WO 2006/055936 A2 describes an automatic external defibrillator with an intelligent self-test system that ensures device readiness. The self-test system conditionally runs functional tests based on knowledge of device use, time of day, pre-programmed information, operational features and previous events. The condition of the defibrillator is indicated visually, audibly or both based on the results of the self-test performed.

US 9,035,787 B2 describes a method, device and/or a system of centralized management and emergency allocation of deployed defibrillators each having associated communication modules. A central server may process a message from a communication module associated with a defibrillator, and then analyze a photograph and/or a video of a defibrillator display to compare it to a set of expected visual markers using a pixel algorithm of a pixel analysis module. The central server may then determine the operational status of the defibrillator such as whether it is in a nonfunctional status.

### Technical Problem

A defibrillator, and a status detection management method and status detection management system therefor are mainly provided in the embodiments according to the present disclosure. The invention is as defined in independent claims 1, 9 and 13-14.

### Solutions to the Problems

### Technical Solutions

According to a first aspect, provided in an embodiment is a status detection management method for a defibrillator, the method comprising:
the defibrillator automatically performing status detection to obtain the current status detection result;
comparing the current status detection result with the last status detection result;
uploading the current status detection result if the current status detection result is different from the last status detection result, and ending the status detection;
determining whether multiple status detection results obtained from multiple times of status detection within a pre-set time period are all the same if the current status detection result is the same as the last status detection result; and
uploading the current status detection result if the multiple status detection results are all the same.

According to a second aspect, provided in an embodiment is a defibrillator, comprising:
a status detection module for automatically performing a status detection on the defibrillator to obtain the current status detection result;
a sending module for sending out the status detection result; and
a processing module for comparing the current status detection result with the last status detection result; notifying the sending module to upload the current status detection result if the current status detection result is different from the last status detection result, and ending the status detection; determining whether multiple status detection results obtained from multiple times of status detection within a pre-set time period are all the same if the current status detection result is the same as the last status detection result; and notifying the sending module to upload the current status detection result if the multiple status detection results are all the same, otherwise not to upload the current status detection result, and ending the status detection.

According to a third aspect, provided in an embodiment is a status detection management system for a defibrillator, the system comprising a terminal for generating a management interface after receiving user login information, the management interface comprises at least a device information area for displaying device information of a plurality of defibrillators associated with the user login information.

According to a fourth aspect, provided in an embodiment is a management device for a defibrillator, the management device comprising a memory and a processor, the processor executes the following processes by calling a program in the memory:
generating a management interface after receiving user login information;
displaying the management interface, the management interface comprises at least a device information area for displaying device information of a plurality of defibrillators associated with the user login information, with the device information comprising at least an device serial number item and a status detection result corresponding to the device serial number;
updating and displaying the status detection result in response to one of the following situations:
   (1) multiple status detection results obtained from multiple times of automatic status detection of the defibrillator within a pre-set time period are the same; and
   (2) two adjacent status detection results of the defibrillator are different.

According to a fifth aspect, provided in an embodiment is a status detection management method for a defibrillator, the method comprising:
the defibrillator performing automatic status detection to obtain the current status detection result;
uploading the current status detection result and ending the status detection in response to one of the following situations:
   (1) multiple status detection results obtained from multiple times of automatic status detection of the defibrillator within a pre-set time period are the same; and
   (2) two adjacent status detection results of the defibrillator are different.

According to a sixth aspect, provided in an embodiment is a defibrillator, comprising: an energy storage circuit, charging and discharging circuits, a wireless communication module, and a controller, wherein
the controller performs automatic status detection on the energy storage circuit and the charging and discharging circuits to obtain the current status detection result, and uploads the current status detection result via the wireless communication module and ends the status detection in response to one of the following situations:
(1) multiple status detection results obtained from multiple times of automatic status detection of the defibrillator within a pre-set time period are the same; and
(2) two adjacent status detection results of the defibrillator are different.

### Beneficial Effects

### Brief Description of the Drawings

### Description of the Drawings

Fig. 1 is a schematic diagram of a system structure of an automatic external defibrillator according to an embodiment;
Fig. 2 is a schematic diagram of the circuit topology of an automatic external defibrillator according to an embodiment;
Fig. 3 is a flow chart of a status detection management method for an automatic external defibrillator according to an embodiment;
Fig. 4 is a flow chart of a status detection management method for an automatic external defibrillator according to another embodiment;
Fig. 5 is a schematic diagram of the topology of a discharging loop with an H-bridge switch circuit of an automatic external defibrillator according to an embodiment;
Fig. 6 is a schematic diagram of the structure of an automatic external defibrillator according to an embodiment;
Fig. 7 is a flow chart of a status detection management method for an automatic external defibrillator according to still another embodiment;
Fig. 8 is a schematic diagram of a device information area displayed on a management interface for a status detection management method according to an embodiment;
Fig. 9 is a schematic diagram of a device management area displayed on a management interface for a status detection management method according to an embodiment;
Fig. 10 is a schematic diagram of a user management area displayed on a management interface for a status detection management method according to an embodiment;
Fig. 11 is a schematic diagram of a device status detection report interface for a status detection management method according to an embodiment; and
Fig. 12 is a schematic diagram of the structure of a status detection management system for an automatic external defibrillator according to an embodiment.

### Embodiments of the present disclosure

### Implementation

### Detailed Description of Embodiments

The embodiments according to the present teaching will be further described in detail below through specific embodiments in conjunction with the accompanying drawings. Associated similar element reference numerals are used for similar elements in various embodiments. In the following embodiments, many details are described so that the present application can be better understood. However, it would be effortlessly appreciated by those skilled in the art that some of the features can be omitted or may be substituted by other elements, materials and methods in each case. In certain cases, some operations involved in the present application are not displayed or described in the specification, which is to prevent the core part of the present application from being obscured by too much description. Moreover, for those skilled in the art, the detailed description of the involved operations is not necessary, and the involved operations can be thoroughly understood according to the description in the specification and the general technical knowledge in the art.

In addition, the characteristics, operations or features described in the specification can be combined in any appropriate manner to form various embodiments. Moreover, the steps or actions in the method description can also be exchanged or adjusted in order in a way that would have been obvious to those skilled in the art. Therefore, the various orders in the specification and drawings are merely for the purpose of clear description of a certain embodiment and are not meant to be a necessary order unless otherwise stated that a certain order must be followed.

The serial numbers themselves for the components herein, for example, "first", "second", etc., are merely configured to distinguish the described objects, and do not have any sequential or technical meaning. Moreover, as used in the present application, "connection" or "coupling", unless otherwise specified, includes both direct and indirect connections (couplings).

It is found that the status detection schemes for the existing automatic external defibrillators have the problem of large energy consumption such as multiple times of defibrillation energy charging and discharging, and also cannot notify the device maintenance personnel of the status detection results in time. An automatic external defibrillator, and a status detection management method, a status detection management system and a device therefor according to the above embodiments optimize the flux consumption of the automatic external defibrillator in the flow of uploading a status detection result, and can reduce the energy consumption of the battery of the automatic external defibrillator when networking and uploading the status detection result. A user can also perform remote management on a plurality of automatic external defibrillators by means of the status detection management system and the device, and allows for timely detection of a machine failure and handle same, thereby effectively avoiding clinical risks.

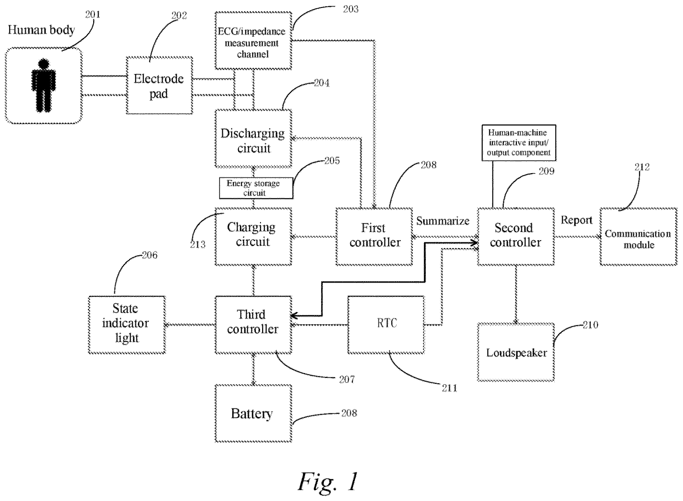

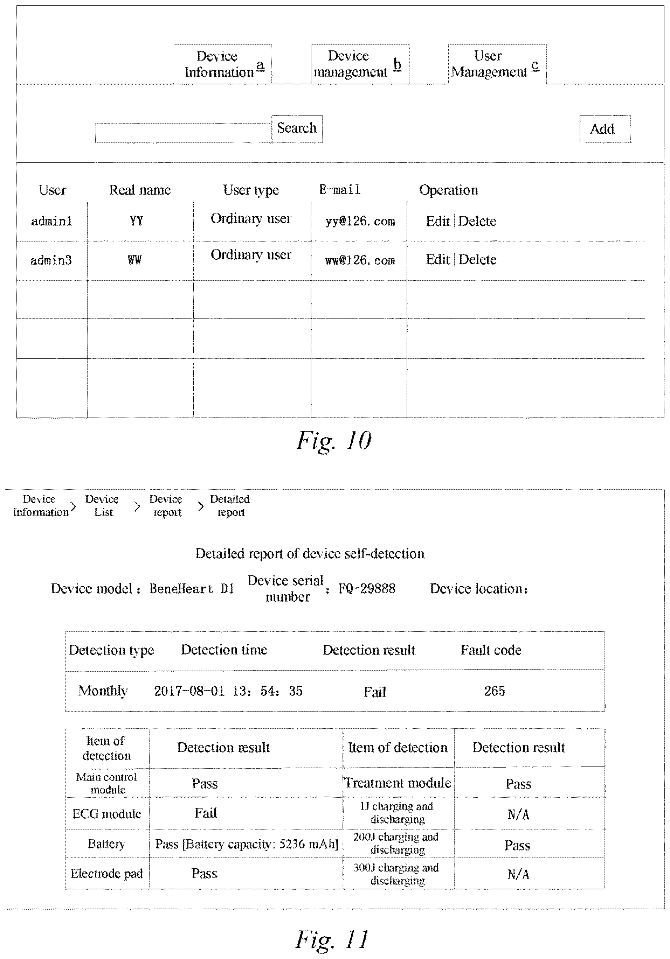

As shown in Fig. 1, a schematic diagram of a system structure of an automatic external defibrillator is provided. In one of the embodiments, defibrillation electrode pads 202 are configured to be attached to the chest surface of a human body 201, and are configured to release a defibrillation current to stimulate the human body. Of course, the electrode pads can also be configured to simultaneously obtain a body surface ECG signal of the human body 201 and impedance information of the chest cavity of the human body between the two electrode pads. What the electrode pads 201 are connected to comprise an ECG/impedance measurement channel 203 and a discharging circuit 204, and both a charging circuit 213 and the discharging circuit 204 are connected to an energy storage circuit 205. A third controller 207 is respectively connected to the charging circuit 213, a state indicator light 206, and a battery 208. The third controller 207 is used for battery and power supply system management, including real-time state information and remaining capacity of a battery, dynamically controlling, according to the current state of the battery, the rate at which the charging circuit charges the energy storage circuit, indicating the lighting operation of the state indicator light 206, etc. The energy storage circuit 205 mentioned herein refers to an energy storage circuit for storing electrical energy for defibrillation, which is a circuit comprising a single element or a combination of multiple elements. The energy storage circuit 205 comprises at least one of a circuit including an energy storage capacitor, a circuit including an energy storage inductor, etc. A first controller 208 is respectively connected to the ECG/impedance measurement channel 203, the discharging circuit 204, and the charging circuit 213, and is configured to control the ECG/impedance measurement channel 203 to measure parameters of the ECG and the impedance of the human body, simultaneously to control the working states of the charging circuit 213 and the discharging circuit 204 to complete the defibrillation charging and discharging operations, and to generate related excitation or control related operations to obtain a status detection execution result of an item of detection from at least one of the ECG/impedance measurement channel 203, the discharging circuit 204, and the charging circuit 213. RTC is a real-time clock circuit 211, which is respectively connected to the third controller 207 and a second controller 209. The RTC can record the status detection time according to clock signals, regularly generate trigger information at a predetermined time, and start a status detection flow including at least one detection item execution process. The RTC generates timing trigger information to the third controller 207. The third controller 207 controls the system such that same is powered on and automatically selects the corresponding status detection flow, including charging the energy storage circuit 205 by the battery through the charging circuit, the first controller 208 obtaining the automatic detection execution results of various items of detection from the ECG/impedance measurement channel 203, the discharging circuit 204 and the charging circuit 213, the second controller 209 obtaining the status detection execution results from the first controller 208 and the third controller 207, storing the status detection results, or/and a communication module 212 outputting the execution results. After the status detection ends, the third controller 207 controls the system such that same is powered down and enters a low-power-consumption sleep mode, and outputs the current status detection execution result via the state indicator light 206. The status detection results can be reported by the communication module 212 to a central management system or a device management platform for unified management and query. In a defibrillator system shown in Fig. 10, the third controller 207 belongs to a main controller of a power supply and battery management system, the first controller 208 belongs to a main controller for an ECG/impedance parameter measurement front end and the defibrillation charging and discharging management, and the second controller 209 belongs to an upper-level controller on a motherboard. In this embodiment, the three controllers belong to different motherboards. Of course, in other embodiments, the three controllers may also be located in different areas of one motherboard, or the three controllers may be integrated and implemented into one controller. The controller mentioned herein may be a processor (CPU or MCU), or the integration of multiple processors (CPUs or MCUs). Therefore, whether the three controllers are either integrated into one controller or are multiple controllers, the method for detecting a defibrillator provided in this embodiment is applicable. For example, in one of the embodiments, the defibrillator is provided with one or more controllers for executing the status detection flow, including at least one detection item execution process, of the defibrillator. The status detection of the automatic external defibrillator includes at least one item of detection. Each item of detection is directed to the detection of a performance of the defibrillator, in which a detection execution result is correspondingly obtained to determine whether the defibrillator is faulty. The item of detection here includes the detection of a hardware circuit performance and the detection of a software performance.

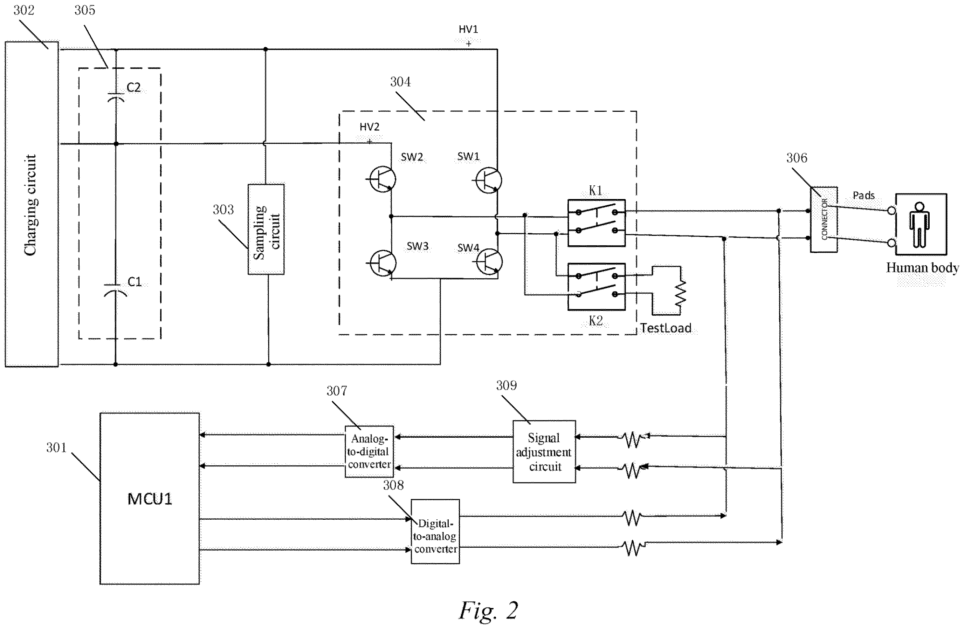

In Fig. 2, the circuit topology of an automatic external defibrillator is provided. As shown in Fig. 2, the defibrillator comprises a controller MCU1, which is labeled 301, a discharging circuit 304, a test load, a charging circuit 302 (same as the charging circuit 213 in Fig. 1), a sampling circuit (voltage sampling circuit as shown in Fig. 2), a battery (like 208 in Fig. 1), and an energy storage circuit 305. The energy storage circuit 305 comprises a capacitor C1 and a capacitor C2, and both the charging circuit 302 and the discharging circuit 304 are connected to the energy storage circuit 305. The energy storage circuit 305 obtains energy from the battery through the charging circuit 302 and stores the energy. Of course, in addition to using the capacitors as an energy storage apparatus in the energy storage circuit 305, other elements can also be used. The discharging circuit 304 comprises an H-bridge switch circuit 304 and a changeover switch circuit. The changeover switch circuit comprises a fifth switch K2 and a sixth switch K1. The H-bridge switch circuit 304 comprises a first switch SW1, a second switch SW2, a third switch SW3, and a fourth switch SW4. The H-bridge circuit is connected in parallel to an output of the energy storage circuit 305, a switch control terminal of the H-bridge circuit is connected to the controller, and the controller outputs a first control signal to control the turning on and off of each switch. The fifth switch K2 and the sixth switch K1 are respectively connected to the test load and the electrode pads for making contact with the human body. The fifth switch K2 and the sixth switch K1 are connected to the output of the H-bridge circuit by respectively connecting midpoints of bridge arms of the H-bridge circuit. The controller outputs a second control signal to control the opening and/or closing of the fifth switch K2 and the sixth switch K1. When the fifth switch K2 and the sixth switch K1 are respectively closed, the test load and the electrode pads are respectively connected to an energizing loop, and communicate with the output of the H-bridge circuit, thereby realizing the discharging operation of the energy storage circuit 305 through the bridge circuit. In some embodiments, the energy storage circuit 305 is configured to lead two inputs with different voltages to voltage input terminals of the H-bridge circuit. In Fig. 2, the energy storage circuit 305 adopts a circuit structure composed of capacitive elements, and of course may also comprise other electronic elements. The test load is a load simulating the impedance of the human body, and is connected in parallel to a human body load terminal via the H-bridge switch circuit 304, and the controller controls the turning on and off of the H-bridge switch circuit 304 to realize the connection of the test load that simulates the impedance of the human body to the energizing loop of the defibrillator. The controller controls the conduction of the charging circuit to realize the power-on operation of the energy storage circuit 305, and the discharging operation of the energy storage circuit 305 is realized by controlling the turning on and off of the H-bridge switch circuit 304 in the discharging circuit. The output of the sampling circuit 303 is connected to two ends of the charging circuit or the output of the energy storage circuit, and is configured to measure the voltage or current output by the energy storage circuit.

In addition, among the circuits, an ECG and impedance channel detection circuit is further comprised, the controller MCU1 is also connected to the energizing loop via a digital-to-analog converter 308, for example, connected to the output terminal of the discharging circuit 304, or the input terminal of a defibrillation electrode 306. In addition, the controller MCU1 controls K1 and K2 to close same at the same time, and the test load is connected in parallel to an input front end of the defibrillation electrode. At the same time, MCU1 outputs excitation signals with different characteristics through the digital-to-analog converter 308 and loads same to the input terminal of the defibrillation electrode, and the controller MCU1 obtains feedback excitation signals from the input terminal of the defibrillation electrode through a signal adjustment circuit 309 and an analog-to-digital converter 307 and carries out detection and processing to detect whether the ECG and impedance measurement channel is normal. The specific indicators of ECG measurement include noise, gain and bandwidth, and the specific indicators of impedance measurement include measurement accuracy.

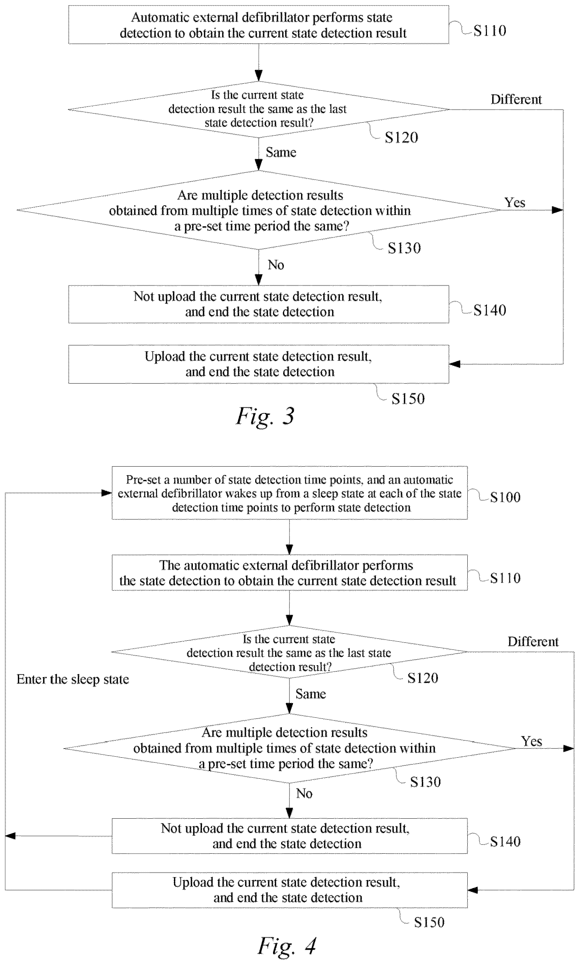

Referring to Fig. 3, in one of the embodiments, a status detection management method for an automatic external defibrillator (hereinafter referred to as a status detection management method) is provided, comprising steps S110 to S150.

Step S 110: the automatic external defibrillator automatically performing status detection to obtain the current status detection result.

Step S120: comparing the current status detection result with the last status detection result; if the current status detection result is different from the last status detection result, proceeding to step S150; and if the current status detection result is the same as the last status detection result, proceeding to step S130.

Step S130: determining whether multiple detection results obtained from multiple times of status detection within a pre-set time period are the same; and if the aforementioned multiple status detection results are all the same, proceeding to step S150, otherwise, proceeding to step S140. In one embodiment, the pre-set time period is 7 days. The term "multiple" mentioned herein refers to two or more.

Step S140: not uploading the current status detection result, and ending the status detection.

Step S150: uploading the current status detection result, and ending the status detection. In one embodiment, step S150 comprises: the automatic external defibrillator activating a wireless communication module to transmit the current status detection result in a wireless communication manner, and deactivating the wireless communication module after the status detection result is transmitted. In one embodiment, step S150 further comprises: continuing to perform the wireless connection for a pre-set number of times, for example 3 times, if the automatic external defibrillator determines that the wireless connection is unsuccessful after activating the wireless communication module, and the automatic external defibrillator storing the current status detection result and entering the sleep state if the connection is still unsuccessful.

Referring to Fig. 4, in one embodiment, the status detection management method may further comprise step S100: pre-setting a plurality of status detection time points, and the automatic external defibrillator waking up from the sleep state at each of the status detection time points to perform the status detection. In one embodiment, the automatic external defibrillator re-enters the sleep state after the status detection ends. In one embodiment, the power-on time may be set to be a status detection time point, and it is also possible to set a certain time of each day to be a status detection time point, a certain time of each week to be a status detection time point, a certain time of each quarter to be a status detection time point, etc.

In one embodiment, different items of detection can be detected at different status detection time points, for example, there may be different items of detection between the status detection performed every day and the status detection performed monthly. Correspondingly, the status detection result comprises the results of a plurality of items of detection of the automatic external defibrillator.

Since the automatic external defibrillator provided in public places is generally provided with a primary battery for power supply, and battery power consumption caused by device networking is the main problem. For example, when the device is in a standby and sleep state, the current is within 1mA, and when the wireless communication module is activated for networking, the current may reach 500 mA or more at the peak power. This will shorten the standby life of the battery, which may lead to the risk of battery power shortage in clinical use. The status detection management method proposed in this embodiment focuses on optimizing the flux consumption of the automatic external defibrillator in the flow of uploading the status detection result. For example, the automatic external defibrillator is in a low-power-consumption sleep state under normal standby, in which the wireless communication module is in a deactivated state. When the status detection needs to be performed, the automatic external defibrillator automatically performs the status detection. In this embodiment, after obtaining the status detection result, not every status detection result is transmitted. Instead, whether to activate the wireless communication module to transmit the status detection result is determined according to the current status detection result and historical status detection results. When the current status detection result is different from the last status detection result, or multiple status detection results obtained from multiple times of status detection within a pre-set time period are all the same, the wireless communication module is activated for networking to transmit the current result for uploading same. After the status detection result is transmitted, the wireless communication module is deactivated again, so that the power consumption of the battery power caused by the networking of the device has been reduced a lot.

In one of the embodiments, in the above step S110, the automatic external defibrillator automatically performs automatic status detection, and the process of obtaining the current status detection result may be implemented as follows.

First, trigger information for triggering the detection of the automatic external defibrillator is obtained;
then, a power-on operation is performed on the defibrillator based on the trigger information;
then, a first detection strategy is automatically executed, the first detection strategy comprising at least performing one of an item of ECG measurement channel detection, an item of discharging loop detection and an item of impedance measurement channel detection; and
finally, the execution result of the item of detection is recorded to obtain the status detection result.

In some embodiments, after automatically executing the first detection strategy, the method further comprises the following steps:
automatically performing a charging operation on the energy storage circuit of the defibrillator within a first time period; and
after charging is completed, automatically executing a second detection strategy within a second time period, and completing a discharging operation of the energy storage circuit within the second time period, the second detection strategy sequentially executes at least one of an item of defibrillation waveform detection and an item of energy release function detection. In one of the embodiments, before or after automatically executing the first detection strategy, the method further comprises: automatically executing an item of battery capability detection.

The trigger information may be obtained based on a timing trigger or an event trigger.

For example, if based on the timing trigger, the defibrillator may have a built-in timing circuit. The timing circuit may be a real-time clock (abbreviation: RTC), and the timing duration of the RTC may be set in advance, which is set to be an interval duration such as every m hours, every n days, every p weeks, or every q months, etc. When the timing duration of the RTC is reached, the defibrillator can be triggered to perform defibrillation status detection. For example, specifically, if the duration of the last detection reaches 5 hours or 2 days, then the defibrillator can perform the defibrillator status detection, and the specific items of detection are not limited in this application. In one of the embodiments, the defibrillator is in a standby state, and the timing clock RTC in the defibrillator is regularly triggered according to a pre-set time rule to generate trigger information, and is configured to send same to the controller to start a status detection flow.

For another example, if the obtaining is based on the event trigger, a power-on trigger and a user input trigger may be mainly comprised. Specifically, a user may input an instruction to the defibrillator. The instruction may be input via a physical key or a virtual key of the defibrillator, or may be input by the user in a remotely controlled manner via a terminal device in communication with the defibrillator. For example, after the defibrillator is powered on, the defibrillator is triggered to perform the status detection on the defibrillator. The specific event triggering means is not limited in this application. In one of the embodiments, the defibrillator further comprises a human-machine interactive input/output component. The trigger information is obtained based on an instruction input by a user via the human-machine interactive input/output component, and is configured to be sent to the controller to start a status detection flow. In one of the embodiments, the defibrillator may also obtain the trigger event delivered from a host computer (or central station) via the communication module, so as to obtain the trigger information, which is configured to be sent to the controller to start the status detection flow.

In one of the embodiments, after receiving the trigger information, the controller of the defibrillator can control the charging circuit to perform a power-on operation on the energy storage circuit of the defibrillator.

Of course, in one of the embodiments, the controller of the defibrillator will automatically start the status detection flow of detecting the defibrillator after receiving the trigger information. The status detection flow here comprises at least one item of detection in relation to the defibrillator.

In some embodiments, the above steps comprise: searching for a status detection strategy according to the trigger information, different trigger information corresponds to different status detection strategies, and automatically starting the power-on operation of the defibrillator based on the status detection strategy. Of course, a status detection strategy formed by the combination of a variety of different items of detection may be stored in the defibrillator in advance. Based on different trigger information, the status detection strategy is different, for example, different items of detection may be executed at different time intervals. Each item of detection is directed to the detection of a performance of the defibrillator, in which a detection execution result is correspondingly obtained to determine whether the defibrillator is faulty. The item of detection here includes the detection of a hardware circuit performance and the detection of a software performance.

Determining the status detection strategy based on the trigger information comprises at least one of the following ways:
A. The status detection strategy is determined according to the interval between a first detection time and a second detection time, the second detection time is earlier than the first detection time, and the first detection time and the second detection time are both detection times triggered regularly.
   For example, detection strategies may be respectively defined for interval durations such as every m hours, every n days, every p weeks, or every q months, etc., and the items of detection corresponding to these timing durations may be partially the same or partially different. Only some of the items of detection may be detected within some of the timing durations, and all the items of detection may be detected within some of the timing durations, which can be specifically set according to the actual use of the defibrillator and other factors, with the aim of reducing the energy loss caused by unnecessary items of detection, without specific limitations thereto in this application.
B. The status detection strategy is determined based on the trigger information obtained by an event trigger mechanism. The event trigger mechanism comprises power-on trigger or user input trigger.

In some embodiments, the status detection strategy comprises detection of at least one detection object, and detection of at least one item of detection of the detection object. The detection object refers to various functional circuits in the defibrillator. The item of detection refers to the various functions or parameters of the detection object.

Specifically, on the premise of ensuring that the detection function of the defibrillator does not affect normal use, in order to reduce unnecessary items of detection and save on energy of the defibrillator, multiple status detection strategies may also be defined. Each status detection strategy corresponds to one or more detection-triggering scenarios.

For example, the power-on trigger and the user input trigger can be respectively defined to correspond to one or more detection strategies. After the defibrillator is powered on, the defibrillator starts the status detection flow of the defibrillator. The started status detection flow of the defibrillator can be performed based on a pre-set detection strategy, which may be dynamically or statically set. The detection object and the item of detection can be updated in the detection strategy according to actual requirements and other factors, and the specific settings thereof are not limited in this application.

By way of example, the detection object that can be detected by the defibrillator mainly includes the first controller, the second controller, the third controller, the battery, a RTC wake-up function, an ECG/impedance channel front end, a basic defibrillation function, a defibrillation discharging detection, a synchronous defibrillation interface, a charging rate IO function, a power supply voltage, etc. The ECG/impedance channel comprises an electrocardiogram (abbreviation: ECG)/ion implantation (impedance, abbreviation: IMP).

In some embodiments, for the status detection strategy determined according to the time interval between the first detection time and the second detection time, different status detection strategies may be automatically started according to different time intervals. For example, the status detection flow of the defibrillator may be set to be status detection strategies in four scenarios: user detection, daily status detection, weekly status detection, and power-on status detection. For the four scenarios, different status detection strategies can be provided, such that different items of detection automatically are started based on different application scenarios, which can effectively reduce the power consumption of the battery of the defibrillator and increase the service life of the battery.

The execution result obtained after the automatic detection comprises a fault code, which is a code configured to indicate that the automatic external defibrillator is faulty.

In some of the embodiments, no matter what the scenario, the controller automatically executes the first detection strategy. The item of ECG measurement channel detection, the item of discharging loop detection and the item of impedance measurement channel detection can be executed in any combination order. When the execution result output from any one of the items of detection is indicative of a fault of the defibrillator, the first detection strategy or the current execution process is terminated, and the process jumps to the step of recording the execution result of the item of detection, for example, recording a fault code. This can effectively reduce the power consumption, optimize the detection order, and shorten the detection time.

In one of the embodiments, the item of ECG measurement channel detection and the item of impedance measurement channel detection mainly detect whether the ECG and IMP measurement channels are normal in a targeted manner, and the two can share a sampling channel, for example, a channel detection circuit part in the circuit topology shown in Fig. 2.

In some embodiments, the item of ECG measurement channel detection and/or the item of impedance measurement channel detection comprise the following steps:
connecting a test load, which simulates the impedance of a human body, to a loop of the defibrillator as a load simulating body contact;
generating an excitation signal at two ends of the test load;
obtaining a sampled signal by detecting the circuit; and
comparing the excitation signal and the sampled signal, determining whether the channel state of the defibrillator is abnormal according to the result of the comparison, and generating an execution result indicating that the defibrillator is faulty when it is determined that the channel state of the defibrillator is abnormal, or executing the next detection step, that is, the next item of detection, when it is determined that the channel state of the defibrillator is normal.

The sampled signal may be a voltage signal obtained by the voltage sampling circuit 303, or may also be an acquired circuit loop current sampled signal. The sampled signal in this embodiment may be the output voltage sampled from the energy storage circuit. ECG channel detection and IMP channel detection will be respectively taken as examples below for description.

As shown in Fig. 2, the test load is a load simulating the impedance of the human body, and is connected in parallel to a human body load terminal via the H-bridge switch circuit 304, and the controller controls the turning on and off of the H-bridge switch circuit 304 to realize the connection of the test load that simulates the impedance of the human body to the loop of the defibrillator.

The H-bridge switch circuit comprises a first switch SW1, a second switch SW2, a third switch SW3, a fourth switch SW4, a fifth switch K2, and a sixth switch K1. The fifth switch K2 and the sixth switch K1 are respectively connected to the test load and the electrode pads for making contact with the human body. The controller outputs a second control signal to control the opening and/or closing of the fifth switch K2 and the sixth switch K1. When the fifth switch K2 and the sixth switch K1 are respectively closed, the test load and the electrode pads are respectively connected to the loop. The controller MCU1 can superimpose the generated excitation signal on two ends of the test load via the digital-to-analog converter 308, and can obtain the output voltage of the energy storage circuit 305 via the voltage sampling circuit 303, to obtain the sampled signal.

As shown in Fig. 2 again, the first switch SW1 and the fourth switch SW4 are connected in series, and the second switch SW2 and the third switch SW3 are connected in series. The fifth switch K1 is configured to control the connection of the test load. The second switch SW2 is electrically connected to one end of the energy storage capacitor C1, one end of the first switch SW1 is electrically connected to one end of the energy storage capacitor C2, and two ends of the sampling circuit are respectively electrically connected to two ends of the energy storage capacitors C1 and C2. When an object to be detected is connected to the defibrillator, the fifth switch K1 is configured to connect the object to be detected. The object to be detected may be other external termination load that simulates the human body.

In some embodiments, after the fifth switch K1 is closed, the test load is turned on, and the H-bridge circuit and the test load form part of an H-bridge discharging loop.

The H-bridge discharging loop comprises a first phase loop and a second phase loop, the first phase loop comprises the first switch, the third switch and the test load, and the second phase loop comprises the second switch, the fourth switch and the test load.

Figs. 1 and 2 are schematic diagrams of the circuit structure of a detection circuit of the defibrillator. Fig. 1 is a schematic diagram when the human body is electrically connected to the defibrillator via the electrode pads. The other loads in Fig. 2 can be replaced with test loads, to carry out the flow of detecting the defibrillator.

In the IMP impedance measurement channel detection, the controller controls the digital-to-analog converter 308 to generate an excitation signal. The controller closes the fifth switch K2 and the sixth switch K1, connects the test load to the circuit, carries out sampling via the analog-to-digital converter 307 to detect the measured impedance value, and compares the measured impedance value with the resistance value of the test load to detect the measurement accuracy of the IMP impedance measurement channel. When the IMP impedance measurement channel is normal, the next step for the next item of detection is proceeded, otherwise an execution result indicating that the defibrillator is faulty is generated. The measured impedance value obtained may be obtained from the ratio of the voltage value and the current value obtained in the sampled signal.

In the ECG measurement channel detection, as shown in Fig. 2, the controller (MCU1) controls the digital-to-analog converter (DAC) 308 to generate an excitation signal, and carries out sampling via the analog-to-digital converter 307 to detect the measurement accuracy of the ECG channel. The specific indicators comprise: noise, gain and bandwidth. Specifically, the digital-to-analog converter DAC is configured to generate an excitation signal, which comprises signals of pre-set values of voltage and current and frequency. The excitation signal generated by the DAC can be added to the forefront end of the entire circuit shown in Fig. 2 and then sampled by the analog-to-digital converter ADC and processed by a series of circuits to obtain the sampled signal. MCU1 compares the excitation signal output by the digital-to-analog converter DAC with the sampled signal sampled by the analog-to-digital converter ADC to determine whether the entire signal processing path is abnormal.

Similarly, when detecting the IMP impedance measurement channel, the controller (MCU1) controls the digital-to-analog converter DAC to generate an excitation signal, and the sampling frequency of the analog-to-digital converter ADC may be the same as the detection of the ECG channel. MCU1 controls K1 and K2 to close same, such that the test load (TestLoad) is connected, that is, TestLoad is electrically connected to PADs, and this is equivalent to that the electrode pads are connected to the human body. TestLoad can simulate the resistance of 50 ohms of the human body. MCU1 obtains the impedance value of the impedance test channel and determines whether the impedance value is within 50 ohms ± 10%. If the impedance value meets this numerical range, MCU1 can determine that the current impedance measurement channel is normal.

The measured impedance value is compared with the impedance value simulated by TestLoad, and MCU1 detects the measurement accuracy of the IMP channel according to the comparison result. When it is determined that the item of ECG/IMP impedance measurement channel detection is normal, the next item of detection can be proceeded. If it is determined that the item of ECG and/or IMP impedance measurement channel detection fails, a fault code is recorded, is sent to the controller, and is then uploaded to a device management system or a host computer server through a communication module of Wifi/4G or another type of the defibrillator. Thereafter, the defibrillator system enters a low-power-consumption standby mode.

The discharging loop detection mainly comprises an item of H-bridge discharging loop detection.

In one of the embodiments, the item of discharging loop detection may comprise: detecting the on-off performance of the discharging loop including the H-bridge switch circuit in the defibrillator, and generating an execution result indicating that the defibrillator is faulty in response to the abnormal on-off performance of the discharging loop. Specifically, the following way may be adopted:
the item of discharging loop detection may specifically comprise:
after the energy of the charging circuit controlled by the controller reaches a first energy value, respectively closing a target switch, which includes one of the following: the first switch, the second switch, the third switch, the fourth switch, or the sixth switch. The numerical interval of the first energy value may be 1J - 2J, or within 100 V, which is not specifically limited in this application.

The controller obtains the loop current after closing the target switch, and determines whether the turning on and off of the target switch are normal according to the corresponding loop current.

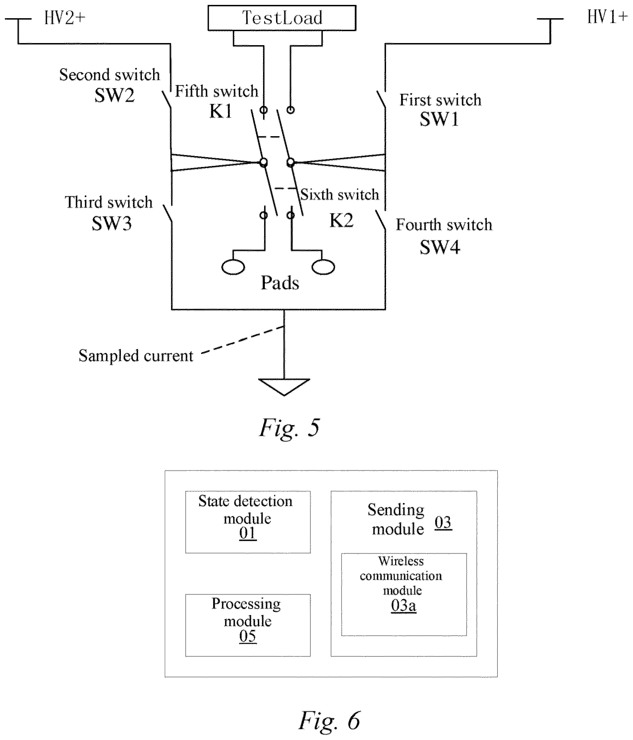

In this application, the item of discharging loop detection mainly detects the on-off performance of the discharging loop including the H-bridge switch circuit. Specifically, Fig. 5 is the topology of a discharging loop with an H-bridge switch circuit. The discharging loop including the H-bridge switch circuit comprises a first switch SW1, a second switch SW2, a third switch SW3, a fourth switch SW4, a fifth switch K1 and a second switch K2. HV1 and HV2 respectively correspond to the corresponding capacitor voltages of a first phase loop and a second phase loop. In one of the embodiments, the controller may perform one of the following operations to detect whether the discharging loop is normal:
the controller can close the first switch to obtain the corresponding first loop current, and determine whether the turning on and off of the first switch are normal according to the first loop current;
the controller closes the second switch to obtain the corresponding second loop current, and determines whether the turning on and off of the second switch are normal according to the second loop current;
the controller closes the third switch to obtain the corresponding third loop current, and determines whether the turning on and off of the third switch are normal according to the third loop current;
the controller closes the fourth switch to obtain the corresponding fourth loop current, and determines whether the turning on and off of the fourth switch are normal according to the fourth loop current; and
the controller closes the second switch, the third switch and the sixth switch to obtain the corresponding fifth loop current, and determines whether the turning on and off of the first switch are normal according to the fifth loop current. The first loop current, the second loop current, the third loop current, the fourth loop current and the fifth loop current can obtain the corresponding loop current at the current sampling position shown in Fig. 2 when using the controller to realize the closing modes mentioned above. In summary, the controller can be configured to sequentially close the switches in the H-bridge switch circuit in a predetermined order to obtain the sampled current in the discharging loop. Whether the turning on and off of the switches in the discharging loop are normal is determined based on the sampled current. In response to the turning on and off of the discharging loop are normal, a step is performed, that is, a next item of detection is performed. On the contrary, in response to the turning on and off of the discharging loop are abnormal, an execution result indicating that the defibrillator is abnormal or faulty is output. In one of the embodiments, once the execution result indicating that the defibrillator is abnormal or faulty is output, the current execution process, that is, the status detection flow is terminated, thereby shortening the status detection flow, and the fault result is directly reported, so that the battery consumption is reduced.

The first detection strategy is not limited to the item of ECG measurement channel detection, the item of discharging loop detection and the item of impedance measurement channel detection, and may also comprise other items of detection.

In the status detection flow, a power-on operation is performed on the defibrillator. In one of the embodiments, the defibrillator is in a standby state before obtaining the trigger information, and waits to start the power-on operation of the defibrillator based on the trigger information. After power-on, a circuit board is energized. At this time, the energy storage circuit for defibrillation charging and discharging is connected to a loop provided by the battery. For example, as shown in Fig. 1, the energy storage circuit 205 will communicate with the battery 208 after the energization operation, and the electric energy provided by the battery 208 will be supplied to the energy storage circuit. In addition, after the power-on operation, the circuit board where the energy storage circuit is located and other circuit boards may all be powered on. In one of the embodiments, the controller in the defibrillator controls, based on the received trigger information, the battery to supply power to the energy storage circuit through the charging circuit.

During the execution of the first detection strategy, the first detection strategy comprises at least one of an item of ECG measurement channel detection, an item of impedance measurement channel detection and an item of discharging loop detection. In one of the embodiments, based on the system structure provided in Fig. 1, the controller in the defibrillator obtains the execution result of the status detection from at least one of the charging loop, the discharging loop and the impedance path when performing the first status detection step. In some embodiments, the item of ECG/impedance measurement channel detection is configured to detect whether the measurement performance of the ECG channel is normal, which is specifically determined by evaluating parameters such as noise, gain and bandwidth of the measurement channel. The item of impedance measurement channel detection is configured to detect whether the measurement accuracy of the impedance of the human body is normal, which can be specifically determined by comparing the impedance value, measured after connecting and simulating the impedance of the human body, with a standard value. The item of ECG/impedance measurement channel detection comprises at least one of ECG channel detection and IMP channel detection. The item of discharging loop detection is configured to detect the on-off performance of the discharging loop.

The recording the execution result of the item of detection comprises: recording the execution result indicating that the defibrillator is faulty. The execution result comprises a fault code, and the execution result mentioned herein is equivalent to the status detection result. Based on the execution result of the item of detection, a fault code is generated, and different items of detection correspond to different fault codes. For different items of detection, different fault codes can be used in the output execution results to indicate the detection output of which item of detection is Faulty, such that the maintenance personnel of a device management platform can understand at a glance what kind of fault has occurred in the defibrillator.

Still further, the status detection strategy obtained based on a certain piece of trigger information may also comprise an item of battery capability detection. For example, the controller automatically executes the item of battery capability detection. In one of the embodiments, before or after automatically executing the first detection strategy, the method further comprises: automatically executing an item of battery capability detection.

In one of the embodiments, the step of automatically executing the battery capability detection comprises at least one of the following steps:
A. detecting the remaining charge level of the battery to determine whether the remaining charge level meets a requirement;
B. reading working state information corresponding to the battery to determine whether the working state information is normal; and
C. causing the battery to realize the defibrillation charging by the charging circuit to determine whether the defibrillation charging can be completed within a specified time; and
generating an execution result indicating that the defibrillator is faulty in response to any of the following situations:
(1) the remaining charge level does not meet the requirement, (2) the working state is abnormal, and (3) the defibrillation charging is not completed within the specified time.

One of or a combination of more than one of the above steps A, B, and C can be selected as the content of the battery capability detection step, and steps A, B, and C are not limited and have no sequential execution order. One of the embodiments is taken as an example below for description.

For example, in one of the embodiments, the automatic execution of the item of battery capability detection may specifically comprise:
the controller obtaining the remaining charge level of the battery, and the controller obtaining the state of a register in the battery if it is determined that the charge level of the battery is not lower than a pre-set charge level according to the remaining charge level. The state of the register may include over-voltage, over-temperature, over-current or other states, and the working state information of the battery is stored in the register. If it is determined that the state of the register is normal, it can be determined that the battery is normal, and the controller performs defibrillation charging on the energy storage capacitor via the battery while the battery is supplying power. When the state of the register may include over-voltage, over-temperature, over-current or other states, it can be determined that the state of the register is abnormal, that is, it can be determined that the battery is abnormal. Optionally, the energy for defibrillation charging may also be set to be pre-set energy.

If the battery completes the defibrillation charging within a pre-set time, the controller may determine that the load capability of the battery is normal. For example, it can be provided that, when being powered, the defibrillator is subjected to the defibrillation charging to reach 360 Joules (J), and if the time it takes to reach 360 J with the defibrillation charging is within a pre-set time, such as 8s, it can be determined that the battery is normal. Correspondingly, the battery capability detection result of the battery may be recorded as Normal, and the defibrillator is not faulty, otherwise, it indicates that the defibrillator is faulty.

Upon the battery capability detection result of the battery being Normal, the next item of detection can be continued, which, for example, may be an item of energy retention characteristic detection of the energy storage circuit, an item of defibrillation biphasic waveform detection, or an item of energy release function detection.

If the battery fails to complete the current defibrillation charging within the pre-set time, the controller may determine that the load capability of the battery is abnormal, indicating that the current detection has failed; and the capability detection result of the battery may be recorded as Faulty, a fault code is marked, an execution result indicating that the defibrillator is faulty is generated, and the process jumps to step 104 to record the execution result. Similarly, the fault code may also be uploaded to the device management system through the built-in communication module of the defibrillator. Correspondingly, the defibrillator enters the low-power-consumption standby mode.

In some embodiments, when the execution of the item of battery capability detection is faulty, the current automatic execution process of detecting the defibrillator may be terminated; and the next item of detection may also be continued, which, for example, may be an item of energy retention characteristic detection of the energy storage circuit, an item of defibrillation biphasic waveform detection, or an item of energy release function detection. This is not specifically limited in this application. From the perspective of reducing the energy consumption, when the detection result of the battery is Faulty, the flow of automatically terminating the detection of the defibrillator is generally selected, such that the defibrillator enters the standby or sleep state.

In one of the embodiments, when it is determined that the execution result output by the first detection strategy indicates that the defibrillator is not faulty, the process of performing the charging operation on the energy storage circuit within the first time period is performed. When the output execution result indicates that the defibrillator is faulty, the current execution process is terminated, and the status detection flow is terminated, thereby shortening the status detection flow, and the process jumps to the step of recording the execution result, so that the battery consumption is reduced.

Still further, in one of the embodiments, in the status detection flow, when it is determined that the execution result output in the battery capability detection step indicates that the defibrillator is not faulty, the first detection strategy is executed, or the charging operation performed on the energy storage circuit is completed. However, when the output execution result indicates that the defibrillator is faulty, the status detection flow is terminated, thereby shortening the status detection flow, and the fault result is directly reported, so that the battery consumption is reduced.

In one of the embodiments, the item of defibrillation waveform detection may comprise:
connecting a test load, which simulates the impedance of a human body, to a loop of the defibrillator, and discharging charge from the energy storage circuit to the test load; acquiring defibrillation current in the loop to obtain a current waveform; and determining whether the current waveform meets a pre-set requirement, and generating an execution result indicating that the defibrillator is faulty in response to the current waveform not meeting the pre-set requirement, otherwise, continuing to execute the next item of detection when the current waveform meets the pre-set requirement, which represents that the detection of the defibrillator is normal this time.

The item of defibrillation waveform detection may comprise one of defibrillation biphasic waveform detection, defibrillation monophasic waveform detection, etc. By way of example, defibrillation biphasic waveform detection will be taken as an example below for description.

The controller closes the fifth switch in Figs. 2 and 5, turns on the test load, and connects the test load to the circuit loop, such that charge is discharged from the energy storage circuit to the test load. The controller respectively performs the following operations:
the controller closing the first phase loop, obtaining a first sampled current waveform sampled by the sampling circuit, and determining whether the difference between the first sampled current waveform and a pre-set first phase loop waveform is within a pre-set range. If the difference between the first sampled current waveform and the pre-set first-phase loop waveform is within the pre-set range, it can be determined that the current first phase loop waveform is normal.

The controller closing the second phase loop, obtaining a second sampled current waveform sampled by the sampling circuit, and determining whether the difference between the second sampled current waveform and a pre-set second phase loop waveform is within the pre-set range. If the difference between the second sampled current waveform and the pre-set second-phase loop waveform is within the pre-set range, it can be determined that the current second phase loop waveform is normal.

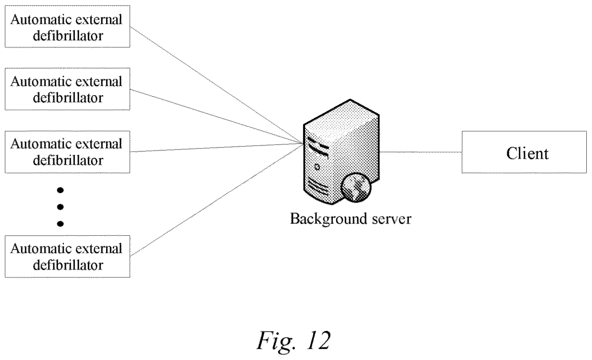

It can be seen that by detecting the first phase loop and the second phase loop separately, the detection of the defibrillation biphasic waveform is realized. It can be seen from Fig. 12 that the defibrillation current can be obtained from the current sampling position in the figure.

By way of example, for example, K2 in Fig. 5 is closed to turn on the test load R; SW1 and SW3 are then closed to turn on the first phase loop composed of SW1, SW3, K2, and the test load R; and the sampled current waveform is then sampled by the ADC to determine whether the shape and parameters of the sampled current waveform are consistent with those of a pre-set waveform. K2 in Fig. 2 is closed to turn on the test load R; SW2 and SW4 are then closed to turn on the second phase loop composed of SW2, SW4, K2, and the test load R; and the sampled current waveform is then sampled by the ADC to determine whether the shape and parameters of the sampled current waveform are consistent with those of a pre-set waveform.

When the detected defibrillation current waveform is consistent with a pre-set waveform, it indicates that the defibrillator is normal, and the execution result of the item of defibrillation waveform detection is not Faulty, otherwise, it represents that the defibrillator is faulty, and the execution result Faulty is output.

In addition, the item of energy release function detection comprises: placing the energy storage circuit in a discharging state, detecting a second energy value (e.g., output voltage value) of the energy storage circuit after a third pre-set time, and generating an execution result indicating that the defibrillator is faulty in response to the second energy value (e.g., output voltage value) not reaching a pre-set threshold. In one of the embodiments, the item of energy release function detection may specifically comprise: the controller closing the second switch (SW2 in Figs. 5 and 2), the fourth switch (SW4 in Figs. 5 and 2) and the fifth switch (K1 in Figs. 5 and 2), to release the power from the energy storage circuit, and detecting whether the voltage of the energy storage circuit is less than a voltage threshold within the third pre-set time, and if so, representing that the energy release is normal and the defibrillator is not faulty. On the contrary, it represents that the energy release is abnormal, the defibrillator is faulty, and an execution result indicating that the defibrillator is faulty is generated.

Before the item of defibrillation waveform detection in the second detection strategy, adding an item of energy retention characteristic detection of the energy storage circuit can comprehensively detect the battery performance and find the cause of the fault, thereby determining the fault before the defibrillation waveform detection. In one of the embodiments, the execution time for the item of energy retention characteristic detection of the energy storage circuit is less than or equal to three seconds.

In one of the embodiments, the maximum voltage value of the energy storage circuit when the first detection strategy is executed is smaller than the maximum voltage value of the energy storage circuit when the second detection strategy is executed. In this embodiment, the item of detection with the most energy consumption is executed later, and the power consumption of the battery can be rationally and effectively arranged in the status detection flow to reduce the power consumption.

In one of the embodiments, the item of energy retention characteristic detection of the energy storage circuit comprises at least one of the following steps:
after completion of charging, the voltage of the energy storage circuit reaching a first voltage, detecting a change in the voltage of the energy storage circuit within a first pre-set time, and
after the energy storage circuit is placed in a discharging state and maintained for a second pre-set time, detecting a first energy storage value of the energy storage circuit; and
generating an execution result indicating that the defibrillator is faulty in response to any of the following situations:
   the voltage change exceeds the first pre-set value; and (2) the first energy storage value is lower than a second pre-set value.

In the item of energy retention characteristic detection of the energy storage circuit, the energy storage circuit mainly includes the following two failure situations: one is that the capacitor voltage/energy retention characteristic is poor, and the leakage speed is fast after charging; and the other is that the error of the energy value of an energy storage element (e.g., capacitor) in the energy storage circuit becomes larger. When at least one of the two situations occurs in the energy storage circuit, the accuracy of the defibrillation energy of the defibrillator will be reduced, thereby affecting the treatment effect of the defibrillator.

In one of the embodiments, in this embodiment, a possible detection scheme is respectively proposed for the two failure situations of the energy storage circuit: during the detection, defibrillation charging is performed on the energy storage circuit, and discharging is controlled in the discharging process. Specifically, the item of energy retention characteristic detection of the energy storage circuit may specifically comprise:
the controller performing defibrillation charging on the energy storage circuit.

Upon the voltage of the energy storage circuit reaching a first voltage, the controller detects a change in the voltage of the energy storage circuit within the first pre-set time.

If the voltage drop of the energy storage circuit within the first pre-set time is not greater than a pre-set voltage drop (i.e., the first pre-set value), it is determined that the stability of the energy storage circuit is normal, otherwise, it is abnormal and there is a fault.

After determining that the stability of the energy storage circuit is normal, the controller controls the discharge of the energy storage circuit to provide energy for the test load, and records a discharging time, i.e. the second pre-set time, during which the voltage of the energy storage circuit is changed from the first voltage to the second voltage.

The controller calculates the actual capacitance value of the energy storage circuit based on the discharging time, the resistance value of the test load, the first voltage, and the second voltage.

If the actual capacitance value exceeds a pre-set capacitance value, the controller determines that the state of the energy storage circuit is normal, otherwise, it is abnormal and there is a fault.

In this embodiment, the first energy storage value is indicated by a capacitance value. Of course, depending on different energy storage elements in the energy storage circuit, different values will be configured to characterize the first energy storage value, for example, it may also be an inductance value, a combined inductance and capacitance characterization value, etc.

Based on the description above, the item of battery capability detection is configured to detect the charging performance, the stored energy retention performance and the discharge performance of the battery, so as to detect the load capability of the battery. The item of energy retention characteristic detection of the energy storage circuit is configured to detect the voltage of the energy storage circuit and the retention characteristic of the stored energy. This item can be configured to detect the power supply endurance and leakage of the energy storage circuit when the energy storage circuit is used for defibrillation discharging of the defibrillator. The energy storage circuit is used for energy storage, allowing the defibrillator to perform defibrillation discharging treatment on a patient, and ensuring that the defibrillator can complete the defibrillation therapy operation of a set energy. A certain amount of defibrillation energy is stored in an energy storage element via the charging circuit, and the defibrillation therapy of the human body is realized by releasing the energy stored in the energy storage element to the body surface, and an energy storage circuit formed by the energy storage element is the energy storage circuit.

The biphasic waveform detection is configured to detect whether the current used for automatically performing the external defibrillation is a normal waveform, so as to ensure that when the defibrillator is connected to the human body, when biphase current is discharged by the energy storage circuit, the body current waveform is obtained, and then accurately compared with the biphasic waveform.

The item of energy release function detection is configured to detect whether the energy storage circuit can be discharged to a certain threshold within a pre-set time, that is, to detect the safety of the energy storage circuit when discharging. If it can be discharged to a certain threshold, it can be determined that the energy storage circuit can release the stored energy below a safe voltage, and then this represents that the energy release function of the energy storage circuit is normal. For example, it can be provided that the energy storage circuit can release the power below 36 volts within a pre-set time, and then this represents the safety performance of the energy storage circuit is normal.

Regardless of the status detection flow formed by the combination of which items of detection, when any one of the items of detection is executed, once the execution result of the item of detection indicates that the defibrillator is faulty, the process jumps to the step of recording the execution result of the item of detection, and the current execution process is thus terminated. The current status detection path is terminated, the defibrillator enters the sleep or standby state, the detection time is effectively shortened, unnecessary power consumption of the battery is avoided, the service life of the battery is prolonged, and the cause of fault is found in time. In some embodiments, the order of the status detection flow may be: sequentially and automatically executing at least one of an item of discharging loop detection, an item of battery capability detection, an item of energy retention characteristic detection of the energy storage circuit, an item of defibrillation waveform detection, and an item of energy release function detection.

In one of the embodiments, when it is determined that the execution result output by the first detection strategy indicates that the defibrillator is not faulty, the process of performing the charging operation on the energy storage circuit within the first time period is performed. When the output execution result indicates that the defibrillator is faulty, the current execution process is terminated, and the status detection flow is terminated, thereby shortening the status detection flow, and the process jumps to the step of recording the execution result, so that the battery consumption is reduced. Still further, in one of the embodiments, in the status detection flow, when it is determined that the execution result output in the battery capability detection step indicates that the defibrillator is not faulty, the first detection strategy is executed, or the charging operation performed on the energy storage circuit is completed. However, when the output execution result indicates that the defibrillator is faulty, the status detection flow is terminated, thereby shortening the status detection flow, and the fault result is directly reported, so that the battery consumption is reduced. In one of the embodiments, in the second detection strategy, the item of defibrillation waveform detection and the item of energy release function detection are sequentially executed in order, and when any one of the items of detection generates an execution result indicating that the defibrillator is faulty, the current execution process is terminated, the process jumps to recording the execution result, the current status detection path is terminated, and the defibrillator enters the sleep or standby state. It is also possible to prompt a fault warning via the defibrillator's own alarm.

Specifically, in some embodiments, the status detection flow is performed according to the following steps: automatically performing a charging operation on the energy storage circuit of the defibrillator within a first time period; after charging is completed, automatically executing an item of energy retention characteristic detection of the energy storage circuit within a second time period; automatically executing an item of defibrillation waveform detection within the second time period; automatically executing an item of energy release function detection within the second time period; and recording the execution result of the item of detection.

After the execution process of each of the items of detection, a step of determining whether the defibrillator is faulty can be added. When one of the items of detection generates an execution result indicating that the defibrillator is faulty, the process directly jumps to the step of recording the execution result, otherwise, the process continues to execute the other items of detection in the status detection flow until the completion of the step of recording the execution result, the process ends, the defibrillator enters the sleep or standby state, and the detection execution result is recorded, and may also be reported to the host computer or device management platform or management system depending on the detection execution result. This optimized flow embodiment can be applied to weekly automatic detection of the defibrillator, and a more simplified flow can be adopted in the daily automatic detection of the defibrillator, so as to ensure that the spontaneous starting of the automatic detection flow of the defibrillator is more flexible, and the detection procedure can also be optimized, thereby shortening the detection time, reducing the power consumption of the battery, and prolonging the service life of the battery.

In the above embodiment, there is another advantage that it is possible to comprehensively detect multiple items of detection of the defibrillator during the completion of one time of charging and discharging, and a more optimized detection flow can be achieved according to the rational arrangement of the detection time. There is no need to repeatedly perform the charging operation on the defibrillator, thereby shortening the detection time and reducing the power consumption of the battery. It can also be understood that the charging operation on the energy storage circuit of the defibrillator is automatically performed once within the first time period or in the entire status detection flow.

Still further, for example, in one of the embodiments, the various steps such as the item of ECG measurement channel detection and/or the item of impedance measurement channel detection, the item of discharging loop detection, the item of battery capability detection, the item of energy retention characteristic detection of the energy storage circuit, the item of defibrillation biphasic waveform detection, and the item of energy release function detection are sequentially performed; and when the execution result output in one of the items of detection indicates that the defibrillator is faulty, the status detection flow is terminated, the defibrillator can enter the standby state, and the process directly jumps to the step of recording the execution result. Otherwise, all the items of detection are executed in sequence. When one of the items is detected and it is determined that this item indicates a fault, the detection of the subsequent item cannot be continued, the entire detection flow is terminated, and a corresponding fault code is generated. This can reduce the energy loss caused by unnecessary detection.

In the minimum status detection flow, on the basis that the complete execution of all the items of direction of main functions can be ensured, the status detection time is shortened to about 10 seconds, and the basic detection items are completed during the process of one time of powering-on and charging of the energy storage circuit with the battery, thereby shortening the detection time period and reducing the power consumption.

In some embodiments, the order of the status detection flow may be: sequentially and automatically executing at least one of an item of discharging loop detection, an item of battery capability detection, an item of energy retention characteristic detection of the energy storage circuit, an item of defibrillation waveform detection, and an item of energy release function detection.

One of the embodiments further discloses an automatic external defibrillator. Referring to Fig. 6, the automatic external defibrillator comprises a status detection module 01, a sending module 03 and a processing module 05.

The status detection module 01 is configured to automatically perform automatic status detection on the automatic external defibrillator to obtain the current status detection result. In one embodiment, the processing module 05 is provided with a plurality of status detection time points, and is configured to wake up the automatic external defibrillator from a sleep state at each of the status detection time points, to notify the status detection module 01 to perform the status detection on the automatic external defibrillator, and then to control the automatic external defibrillator to enter the sleep state when the status detection ends. In one embodiment, the processing module 05 may set the power-on time to be a status detection time point, and it is also possible to set a certain time of each day to be a status detection time point, a certain time of each week to be a status detection time point, a certain time of each quarter to be a status detection time point, etc.

The sending module 03 is configured to transmit the status detection result. In one embodiment, the sending module 03 comprises a wireless communication module 03a, and the processing module 05 activates the wireless communication module 03a to transmit the current status detection result in a wireless communication manner when the status detection result needs to be sent, and deactivates the wireless communication module 03a after the status detection result is transmitted. In one embodiment, after the processing module 03 activates the wireless communication module 03a, the processing module 03 controls the wireless communication module 03a to continue to perform the wireless connection for a pre-set number of times, for example 3 times, if the wireless connection of the wireless communication module 03a is unsuccessful, and the processing module 05 controls the automatic external defibrillator to store the current status detection result and enter the sleep state if the connection is still unsuccessful.

The processing module 05 is configured to compare the current status detection result with the last status detection result; to notify the sending module 03 to upload the current status detection result if the current status detection result is different from the last status detection result, and end the status detection; to determine whether all the status detection results obtained only within a pre-set time period are the same if the current status detection result is the same as the last status detection result; and to notify the sending module 03 to upload the current status detection result if all the status detection results are all the same within a time period exceeding the pre-set time period, otherwise not to send the current status detection result, and end the status detection. In one embodiment, the pre-set time period is 7 days.

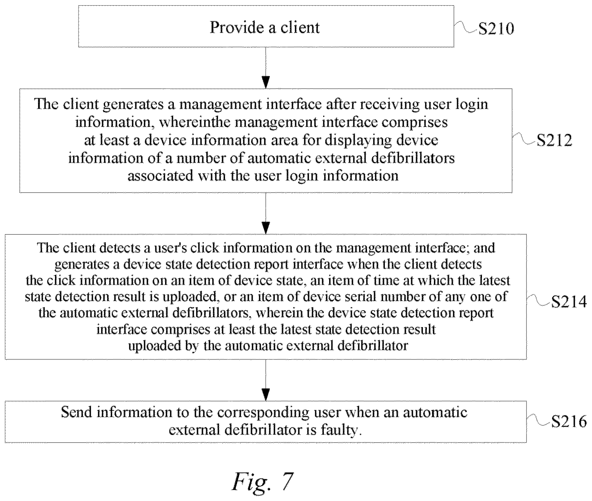

In order to have the ability to remotely and collectively manage multiple automatic external defibrillators and master the state of each automatic external defibrillator in a timely manner, referring to Fig. 7, in one embodiment, the status detection management method may further comprise steps S210 to S216.

Step S210: providing a terminal.

Step S212: the terminal generating a management interface after receiving user login information, the management interface comprises at least a device information area for displaying device information of a plurality of automatic external defibrillators associated with the user login information. In one embodiment, the device information of any one of the automatic external defibrillators comprises at least one of an device state item of the automatic external defibrillator, an latest uploading time item of the status detection result, an device serial number item, a device location item, a maintenance state item, an associated item of user information, and an upgrade reminder item. For example, when a first user logs in, the device information of the number of automatic external defibrillators associated with the first user is displayed, and when a second user logs in, the device information of the number of automatic external defibrillators associated with the second user is displayed.

Step S214: the terminal detecting a clicking of a user on the management interface; and generating a device status detection report interface when the terminal detects the clicking on the device state item, the latest uploading time item of the status detection result, or the device serial number item of any one of the automatic external defibrillators, the device status detection report interface comprises at least the latest status detection result uploaded by the automatic external defibrillator.

Step S216: sending information to the corresponding user when an automatic external defibrillator is faulty. In one embodiment, the status detection result comprises the results of a plurality of items of detection of the automatic external defibrillators, and when any one of the results of the items of detection of any one of the automatic external defibrillators is Faulty, the terminal sends the status detection result to a user associated with the automatic external defibrillator. In one embodiment, when the terminal has not received the status detection result of a certain automatic external defibrillator beyond the pre-set time period, the terminal sends a reminder message to the user associated with the automatic external defibrillator.

In one embodiment, the management interface further comprises a device management area for allowing the user to upgrade a program of the automatic external defibrillator in a wireless manner.

In one embodiment, the management interface further comprises a user management area for managing the user associated with the respective user login information.

Let us take an example to explain how the status detection management method of this embodiment remotely manages multiple automatic external defibrillators.

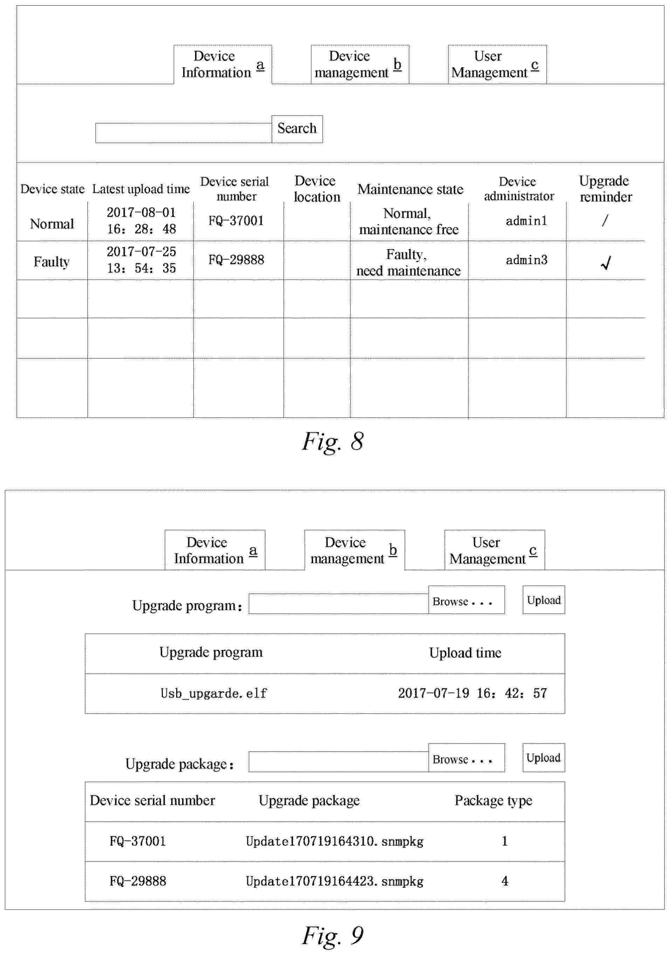

After the terminal receives the user login information, a management interface is generated. The management interface may comprise a device information area, a device management area, a user management area, etc. The display of each area can be switched by clicking on a device information click item a, a device management click item b and a user management click item c on the management interface. When the management interface detects a click on the device information click item a, the management interface then displays the device information area, as shown in Fig. 8. Similarly, when the management interface detects a click on the device management click item b, the management interface then displays the device management area, as shown in Fig. 9. When the management interface detects a click on the user management click item c, the management interface then displays the user management area, as shown in Fig. 10. In one embodiment, the terminal generates a management interface after receiving user login information, and a default display area thereof is the device information area.

Taking the device information area displayed in the management interface in Fig. 8 as an example, it may comprise displaying device information of one or more automatic external defibrillators associated with user login information, comprising displaying an device state item indicating that whether the automatic external defibrillator is normal, faulty or without information, an latest uploading time item of the status detection result, an device serial number item, a device location item, a maintenance state item, an associated item of user information, an upgrade reminder item, etc. A device status detection report interface, as shown in Fig. 11, is generated when the terminal detects the clicking on the device state item, the latest uploading time item of the status detection result, or the device serial number item of any one of the automatic external defibrillators, the content in the device status detection report interface may comprise a device model, a serial number, location, etc., as well as the latest-uploaded status detection result.

As shown in Fig. 9, the user can upgrade, in a wireless manner, the program of the automatic external defibrillator by operating in the device management area. For example, only by selecting the program that needs to be upgraded and the automatic external defibrillator that needs to be upgraded, the program of the selected automatic external defibrillator can be upgraded.

As shown in Fig. 10, the user can manage the users associated with the respective user login information by operating in the user management area. The user management area can display nickname, real name, email address, etc. of each user, and the user can add, delete or edit various information of the user, such as the nickname, real name, email address, etc. of the user mentioned above. In one embodiment, users with different permissions can be set, such as ordinary users and super users. After ordinary users log in, they can view in the user management area, but they cannot perform operations such as adding, deleting or editing, whereas after super users log in, they can not only view, but can also perform operations such as adding, deleting or editing. The terminal can determine whether the user is an ordinary user or a super user based on the user login information.

When any one of the results of the items of detection of any one of the automatic external defibrillators is Faulty, the terminal sends the status detection result to the user associated with the automatic external defibrillator, for example, sending an email to the user's email box. When the terminal has not received the status detection result of a certain automatic external defibrillator beyond the pre-set time period, the terminal sends reminder information to the user associated with the automatic external defibrillator, for example, sending an email to the user's email box, sending a text message to the user's mobile phone, etc.

In one of the embodiments, further disclosed is a status detection management system for an automatic external defibrillator (hereinafter referred to as a status detection management system), which can enable a user to remotely and collectively manage multiple automatic external defibrillators, and master the state of each automatic external defibrillator in a timely manner. In one embodiment, as shown in Fig. 12, the status detection management system may further comprise a background server and/or the automatic external defibrillator disclosed in the embodiments, the background server is configured to receive the status detection result sent by each automatic external defibrillator and allow the terminal to call the status detection result.

The status detection management system comprises a terminal for generating a management interface after receiving user login information, the management interface comprises at least a device information area for displaying device information of a plurality of automatic external defibrillators associated with the user login information. In one embodiment, the device information of any one of the automatic external defibrillators comprises at least one of an device state item of the automatic external defibrillator, an latest uploading time item of the status detection result, an device serial number item, a device location item, a maintenance state item, an associated item of user information, and an upgrade reminder item. In one embodiment, the terminal detects a clicking of a user on the management interface; and generates a device status detection report interface when the terminal detects the clicking on the device state item, the latest uploading time item of the status detection result, or the device serial number item of any one of the automatic external defibrillators, the device status detection report interface comprises at least the latest status detection result uploaded by the automatic external defibrillator. In one embodiment, the status detection result comprises the results of a plurality of items of detection of the automatic external defibrillators, and when any one of the results of the items of detection of any one of the automatic external defibrillators is Faulty, the terminal sends the status detection result to a user associated with the automatic external defibrillator; and/or, when the terminal has not received the status detection result of a certain automatic external defibrillator beyond the pre-set time period, the terminal sends reminder information to the user associated with the automatic external defibrillator.

In order to facilitate the user in performing management such as remote upgrading on the device, in one embodiment, the management interface further comprises a device management area for allowing the user to upgrade a program of the automatic external defibrillator in a wireless manner.

In one embodiment, the management interface further comprises a user management area for managing the user associated with the respective user login information.

Based on the above method and system, in one of the embodiments, further provided is a management device for an automatic external defibrillator, the management device comprising a memory and a processor, the processor executes the following processes by calling a program in the memory:
generating a management interface after receiving user login information;
displaying the management interface, the management interface comprises at least a device information area for displaying device information of a plurality of automatic external defibrillators associated with the user login information, with the device information comprising at least an device serial number item and a status detection result corresponding to the device serial number;
updating and displaying the status detection result in response to one of the following situations: multiple status detection results obtained from multiple times of status detection of the automatic external defibrillator within a pre-set time period are the same; and two adjacent status detection results of the automatic external defibrillator are different.

Based on the above description, in one of the embodiments, further provided is a status detection management method for an automatic external defibrillator, the method comprising:
the automatic external defibrillator performing status detection to obtain the current status detection result;
uploading the current status detection result and ending the status detection in response to one of the following situations: (1) multiple status detection results obtained from multiple times of status detection of the automatic external defibrillator within a pre-set time period are the same; and (2) two adjacent status detection results of the automatic external defibrillator are different.

Based on the above description, in one of the embodiments, further provided is an automatic external defibrillator, comprising: an energy storage circuit, charging and discharging circuits, a wireless communication module, and a controller, wherein
the controller performs status detection on the energy storage circuit and the charging and discharging circuits to obtain the current status detection result, and uploads the current status detection result via the wireless communication module and ends the status detection in response to one of the following situations: (1) multiple status detection results obtained from multiple times of status detection of the automatic external defibrillator within a pre-set time period are the same; and (2) two adjacent status detection results of the automatic external defibrillator are different.

Regarding the process of status detection of the automatic external defibrillator in the above processes, the process of determining multiple status detection results obtained from multiple times of status detection of the automatic external defibrillator within a pre-set time period, the process of determining two adjacent status detection results of the automatic external defibrillator, the management interface, the device information, the status detection result, etc., please refer to the detailed description of the relevant steps hereinabove for specific details. The status detection result in the embodiments comprises a fault code, which can be referred to the related description hereinabove.

The automatic external defibrillator, and the status detection management method and status detection management system therefor provided in the embodiments can reduce the energy consumption of the battery in the process of AED networking and status detection result uploading; moreover, with the status detection management system, the device management personnel can manage multiple AEDs to find and deal with machine faults in a timely manner, which can effectively avoid clinical risks. If necessary, the status detection management system can also support remote software upgrading and GPS positioning of AED devices.

Those skilled in the art would have understood that all or some of the functions of the various methods in the above embodiments may be implemented by means of hardware or by means of a computer program. When all or some of the functions in the above embodiments are implemented by means of a computer program, the program may be stored in a computer-readable storage medium, and the storage medium may include: a read-only memory, a random access memory, a magnetic disk, an optical disk, a hard disk, etc., and the program is executed by a computer to achieve the above functions. For example, the program is stored in a memory of the device, and when the program in the memory is executed by the processor, all or some of the above functions can be implemented. In addition, when all or some of the functions in the above embodiments are implemented by means of a computer program, the program may also be stored in a storage medium such as a server, another computer, a magnetic disk, an optical disk, a flash disk or a mobile hard disk, may be saved to memory of the local device by downloading or copying, or may perform version updating on the system of the local device. When the program in the memory is executed by the processor, all or some of the functions in the above embodiments can be implemented.

The embodiments have been described in detail with reference to specific examples, which are merely for the purpose of facilitating learning about the embodiments and are not intended to limit the embodiments. It will be apparent to those skilled in the art that changes may be made to the specific embodiments described above in accordance with the teachings of the embodiments. The present invention is set out in the claims that follow.

## Claims

1. A status detection management method for a defibrillator, comprising:
automatically performing a status detection (S110), by the defibrillator, to obtain a current status detection result;
comparing (S120) the current status detection result with a last status detection result;
uploading (S150) the current status detection result and ending the status detection, when the current status detection result is different from the last status detection result;
determining (S140) whether multiple status detection results obtained from multiple times of status detection within a pre-set time period are all the same when the current status detection result is the same as the last status detection result; and
uploading (S150) the current status detection result when the multiple status detection results are all the same.

2. The status detection management method of claim 1, further comprising: pre-setting (S100) a plurality of status detection time points, and waking up the defibrillator from a sleep state at each of the status detection time points to perform status detection and re-entering the sleep state after the status detection ends.

3. The status detection management method of claim 1, **characterized in that** the step of uploading the current status detection result comprises: activating a wireless communication module, by the defibrillator, to transmit the current status detection result in a wireless communication manner, and deactivating the wireless communication module after the status detection result is transmitted.

4. The status detection management method of claim 3, further comprising: continuing to perform a wireless connection for a pre-set number of times when the defibrillator determines that the wireless connection is unsuccessful after activating the wireless communication module; and storing the current status detection result and causing the defibrillator to enter into a sleep state when the wireless connection is still unsuccessful.

5. The status detection management method of any one of claims 1-4, further comprising:
providing a terminal; and
generating a management interface by the terminal after receiving user login information, the management interface comprises a device information area for displaying device information of a plurality of defibrillators associated with the user login information;
the device information of any one of the defibrillators comprises at least one of a device state item, a latest uploading time item of a status detection result, a device serial number item, a device location item, a maintenance state item, an associated user information item, and an upgrade reminder item.

6. The status detection management method of claim 5, further comprising: detecting, by the terminal, clicking on the management interface; and generating a device status detection report interface when the clicking of the device state item of any one of the defibrillators, the latest uploading time item of the status detection result, or the device serial number item is detected by the terminal, the device status detection report interface comprises at least the status detection result uploaded lately by the defibrillator.

7. The status detection management method of claim 6, **characterized in that** the status detection result comprises testing results of the defibrillators, and, further comprising: sending, by the terminal, the status detection result to a user associated with one of the defibrillators, when any one of the testing results of the one of the defibrillators is faulty; and/or, sending, by the terminal, a reminder message to the user, when the terminal has not received the status detection result of the one of the defibrillators beyond a pre-set time period.

8. The status detection management method of claim 5, **characterized in that** the management interface further comprises a device management area for allowing a user to upgrade a program of the defibrillator in a wireless manner; and/or
the management interface further comprises a user management area for managing users associated with user login information.

9. A defibrillator, comprising:
a status detection module (01) for automatically performing status detection on the defibrillator to obtain a current status detection result;
a sending module (03) for sending out the current status detection result; and
a processing module (5) for comparing the current status detection result with the last status detection result; notifying the sending module (03) to upload the current status detection result when the current status detection result is different from the last status detection result, and ending the status detection; determining whether multiple status detection results obtained from multiple times of status detection within a pre-set time period are all the same when the current status detection result is the same as the last status detection result; and notifying the sending module (03) to upload the current status detection result if the multiple status detection results are all the same, otherwise not to upload the current status detection result, and ending the status detection.

10. The defibrillator of claim 9, **characterized in that** the processing module (05) is provided with a plurality of status detection time points, and is configured to wake up the defibrillator from a sleep state at each of the status detection time points, to notify the status detection module (01) to perform the status detection on the defibrillator, and then to control the defibrillator to enter the sleep state when the status detection ends.

11. The defibrillator of claim 9, **characterized in that** the sending module (03) comprises a wireless communication module (03a), and the processing module (05) activates the wireless communication module (03a) to transmit the current status detection result in a wireless communication manner when the status detection result needs to be uploaded, and deactivates the wireless communication module after the status detection result is transmitted.

12. The defibrillator of claim 11, after the processing module (05) activates the wireless communication module (03a), the processing module (05) controls the wireless communication module (03a) to continue to perform a wireless connection for a pre-set number of times when the wireless connection of the wireless communication module (03a) is unsuccessful, and the processing module (05) controls the defibrillator to store the current status detection result and enter a sleep state when the wireless connection is still unsuccessful.

13. A management device for a defibrillator, comprising a memory and a processor, the processor executes the following processes by calling a program in the memory:
generating a management interface (S212) after receiving user login information;
displaying (S214) the management interface, the management interface comprises at least a device information area for displaying device information of a plurality of defibrillators associated with the user login information, with the device information comprising at least a device serial number item and a status detection result corresponding to the device serial number;
updating and displaying the status detection result in response to one of the following situations:
(1) multiple status detection results obtained from multiple times of status detections of the defibrillator within a pre-set time period are the same; and
(2) two adjacent status detection results of the defibrillator are different.

14. A computer program comprising instructions which, when the program is executed by a computer, cause the computer to control a defibrillator according to any of claims 9-12 to execute the computer program comprising the status detection management method according to claims 1-8.

## Patentansprüche

1. Zustandserfassungs-Verwaltungsverfahren eines Defibrillators, das Folgendes umfasst:
automatische Durchführung einer Zustandserfassung (SI 10) durch den Defibrillator, um ein aktuelles Zustandserfassungsergebnis zu erhalten;
Vergleichen (S120) des aktuellen Zustandserfassungsergebnisses mit einem zuletzt erfassten Zustandsergebnis;
Hochladen (S150) des aktuellen Zustandserfassungsergebnisses und Beenden der Zustandserfassung, wenn sich das aktuelle Zustandserfassungsergebnis von dem zuletzt erfassten Zustandsergebnis unterscheidet;
Bestimmen (S 140), ob mehrere Zustandserfassungsergebnisse, die aus verschiedenen Zeiten der Zustandserfassung innerhalb einer vorgegebenen Zeitspanne erhalten wurden, alle gleich sind, wenn das aktuelle Zustandserfassungsergebnis gleich ist wie das zuletzt erfasste Zustandsergebnis; und
Hochladen (S150) des aktuellen Zustandserfassungsergebnisses, wenn die verschiedenen Zustandserfassungsergebnisse alle gleich sind.

2. Das Zustandserfassungs-Verwaltungsverfahren nach Anspruch 1 umfasst ferner: Voreinstellen (S100) einer Vielzahl von Zustandserfassungszeitpunkten und Wiedereinschalten des Defibrillators aus einem Ruhemodus zu jedem der Zustandserfassungszeitpunkte, um eine Zustandserfassung durchzuführen, und Wiedereintreten in den Ruhemodus nach Abschluss der Zustandserfassung.

3. Zustandserfassungs-Verwaltungsverfahren nach Anspruch 1, **dadurch gekennzeichnet, dass** der Schritt des Hochladens des aktuellen Zustandserfassungsergebnisses Folgendes umfasst: Aktivieren eines kabellosen Kommunikationsmoduls durch den Defibrillator, um das aktuelle Zustandserfassungsergebnis auf eine kabellose Kommunikationsweise zu übertragen, und Deaktivieren des kabellosen Kommunikationsmoduls, nachdem das Zustandserfassungsergebnis übertragen wurde.

4. Zustandserfassungs-Verwaltungsverfahren nach Anspruch 3, ferner umfassend: Fortsetzen der Durchführung einer kabellosen Verbindung für eine voreingestellte Anzahl von Zeitpunkten, wenn der Defibrillator feststellt, dass die kabellose Verbindung nach der Aktivierung des kabellosen Kommunikationsmoduls nicht erfolgreich ist; und Speichern des Ergebnisses der Erfassung des aktuellen Zustands und Veranlassen des Defibrillators, in einen Ruhemodus einzutreten, wenn die kabellose Verbindung weiterhin nicht erfolgreich ist.

5. Zustandserfassungs-Verwaltungsverfahren nach einem der Ansprüche 1-4, ferner umfassend:
Bereitstellung eines Terminals; und
Erstellen einer Verwaltungsschnittstelle durch das Terminal nach dem Empfang von Benutzeranmeldeinformationen, wobei die Verwaltungsschnittstelle einen Geräteinformationsbereich zur Anzeige von Geräteinformationen einer Vielzahl von Defibrillatoren umfasst, die mit den Benutzeranmeldeinformationen verbunden sind;
Geräteinformationen eines der Defibrillatoren über mindestens eines der folgenden Elemente, die Folgendes umfassen: ein Element für den Gerätezustand, ein Element für die letzte Hochladezeit eines Zustandserfassungsergebnisses, ein Element für die Geräteseriennummer, ein Element für den Gerätestandort, ein Element für den Wartungszustand, ein Element für die zugehörigen Benutzerinformationen und ein Element für eine Aktualisierungsmahnung.

6. Zustandserfassungs-Verwaltungsverfahren nach Anspruch 5, ferner umfassend: Erfassen über das Terminal durch das Anklicken der Verwaltungsschnittstelle; und Erstellen einer Schnittstelle für einen Gerätezustandserfassungsbericht, wenn das Anklicken des Elementes für den Gerätezustand eines der Defibrillatoren, des Elementes für die letzte Hochladezeit des Zustandserfassungsergebnisses oder des Elementes für die Geräteseriennummer durch das Terminal erfasst wird, wobei die Schnittstelle für den Gerätezustandserfassungsbericht mindestens das zuletzt durch den Defibrillator hochgeladene Zustandserfassungsergebnis beinhaltet.

7. Zustandserfassungs-Verwaltungsverfahren nach Anspruch 6, **dadurch gekennzeichnet, dass** das Zustandserfassungsergebnis Testergebnisse der Defibrillatoren und außerdem Folgendes umfasst: Senden des Zustandserfassungsergebnisses durch das Terminal an einen Benutzer, der mit einem der Defibrillatoren verbunden ist, wenn eines der Testergebnisse eines der Defibrillatoren fehlerhaft ist; und/oder Senden einer Aufforderung durch das Terminal an den Benutzer, wenn das Terminal das Zustandserfassungsergebnis eines der Defibrillatoren nach einer voreingestellten Zeitspanne nicht erhalten hat.

8. Zustandserfassungs-Verwaltungsverfahren nach Anspruch 5, **dadurch gekennzeichnet, dass** die Verwaltungsschnittstelle ferner einen Geräteverwaltungsbereich umfasst, der es einem Benutzer ermöglicht, ein Programm des Defibrillators auf kabellose Weise zu aktualisieren; und/oder
die Verwaltungsschnittstelle ferner einen Benutzerverwaltungsbereich beinhaltet, um mit Benutzeranmeldeinformationen verbundene Benutzer zu verwalten.

9. Ein Defibrillator, mit:
einem Zustandserfassungsmodul (01) zur automatischen Durchführung der Zustandserfassung des Defibrillators, um ein Ergebnis der aktuellen Zustandserfassung zu erhalten;
einem Sendemodul (03) zum Senden des Ergebnisses der aktuellen Zustandserfassung; und
einem Verarbeitungsmodul (5) zum Vergleichen des aktuellen Zustandserfassungsergebnisses mit dem zuletzt erfassten Zustandserfassungsergebnis; zur Benachrichtigung des Sendemoduls (03), um das aktuelle Zustandserfassungsergebnis hochzuladen, wenn sich das aktuelle Zustandserfassungsergebnis von dem zuletzt erfassten Zustandsergebnis unterscheidet, und zum Abschließen der Zustandserfassung; zur Bestimmung, ob mehrere Zustandserfassungsergebnisse, die aus mehreren Zeitpunkten der Zustandserfassung innerhalb einer voreingestellten Zeitspanne erhalten wurden, alle gleich sind, wenn das aktuelle Zustandserfassungsergebnis das gleiche ist wie das zuletzt erfasste Zustandsergebnis; und zur Mitteilung an das Sendemodul (03), das aktuelle Zustandserfassungsergebnis hochzuladen, wenn die mehreren Zustandserfassungsergebnisse alle gleich sind, andernfalls das aktuelle Zustandserfassungsergebnis nicht hochzuladen, und die Zustandserfassung abzuschließen.

10. Defibrillator nach Anspruch 9, **dadurch gekennzeichnet, dass** das Verarbeitungsmodul (05) mit einer Vielzahl von Zustandserfassungszeitpunkten versehen ist und so ausgelegt ist, dass es den Defibrillator zu jedem der Zustandserfassungszeitpunkte aus einem Ruhemodus aufweckt, das Zustandserfassungsmodul (01) benachrichtigt, die Zustandserfassung am Defibrillator durchzuführen, und dann den Defibrillator so steuert, dass er in den Ruhemodus übergeht, sobald die Zustandserfassung abgeschlossen ist.

11. Defibrillator nach Anspruch 9, **dadurch gekennzeichnet, dass** das Sendemodul (03) ein kabelloses Kommunikationsmodul (03a) umfasst, und das Verarbeitungsmodul (05) das kabellose Kommunikationsmodul (03a) aktiviert, um das aktuelle Zustandserfassungsergebnis in einer kabellosen Kommunikationsweise zu übertragen, wenn das Zustandserfassungsergebnis hochgeladen werden muss, und das kabellose Kommunikationsmodul deaktiviert, nachdem das Zustandserfassungsergebnis übertragen wurde.

12. Defibrillator nach Anspruch 11, wobei nach der Aktivierung des kabellosen Kommunikationsmoduls (03a) durch das Verarbeitungsmodul (05) das kabellose Kommunikationsmodul (03a) durch das Verarbeitungsmodul (05) so gesteuert wird, dass es weiterhin eine kabellose Verbindung für eine voreingestellte Häufigkeit durchführt, wenn die kabellose Verbindung des kabellosen Kommunikationsmoduls (03a) nicht erfolgreich ist, und wobei das Verarbeitungsmodul (05) den Defibrillator so steuert, dass es das aktuelle Zustandserfassungsergebnis speichert und in einen Ruhemodus übergeht, falls die kabellose Verbindung weiterhin nicht erfolgreich ist.

13. Verwaltungsvorrichtung für einen Defibrillator mit einem Speicher und einem Prozessor, wobei der Prozessor durch den Aufruf eines Programms im Speicher folgende Verfahren ausführt:
Erstellen einer Verwaltungsschnittstelle (S212) nach Erhalt von Benutzeranmeldeinformationen;
Anzeigen (S214) der Verwaltungsschnittstelle, wobei diese mindestens einen Geräteinformationsbereich zur Anzeige von Geräteinformationen einer Vielzahl von Defibrillatoren umfasst, die mit den Benutzeranmeldeinformationen verbunden sind, wobei die Geräteinformationen mindestens ein Element mit einer Geräteseriennummer und ein der Geräteseriennummer entsprechendes Zustandserfassungsergebnis beinhalten;
Aktualisieren und Anzeigen des Zustandserfassungsergebnisses als Reaktion auf eine der folgenden Situationen:
(1) wenn mehrere Zustandserfassungsergebnisse, die aus mehreren Zustandserfassungen des Defibrillators innerhalb eines vorgegebenen Zeitraums gewonnen werden, gleich sind; und
(2) zwei aufeinander folgende Zustandserfassungsergebnisse des Defibrillators unterschiedlich sind.

14. Computerprogramm mit Anweisungen, die, wenn das Programm von einem Computer ausgeführt wird, ihn dazu veranlassen, einen Defibrillator nach einem der Ansprüche 9-12 zu steuern, um das Computerprogramm mit dem Zustandserfassungs-Verwaltungsverfahren nach den Ansprüchen 1-8 auszuführen.

## Revendications

1. Procédé de gestion de détection d'état pour un défibrillateur, comprenant les étapes consistant à :
effectuer automatiquement une détection d'état (S110), via le défibrillateur, pour obtenir un résultat de détection d'état actuel ;
comparer (S120) le résultat de détection d'état actuel à un dernier résultat de détection d'état ;
charger (S150) le résultat de détection d'état actuel et mettre fin à la détection d'état, lorsque le résultat de détection d'état actuel est différent du dernier résultat de détection d'état ;
déterminer (S140) si de multiples résultats de détection d'état obtenus de multiples instants de détection d'état dans un laps de temps prédéfini sont tous les mêmes lorsque le résultat de détection d'état actuel est le même que le dernier résultat de détection d'état ; et
charger (S150) le résultat de détection d'état actuel lorsque les multiples résultats de détection d'état sont tous les mêmes.

2. Procédé de gestion de détection d'état selon la revendication 1, comprenant en outre : le fait de prédéfinir (S100) une pluralité d'instants de détection d'état, et de sortir le défibrillateur d'un état de veille à chacun des instants de détection d'état pour effectuer une détection d'état et de le remettre en état de veille une fois que la détection d'état est terminée.

3. Procédé de gestion de détection d'état selon la revendication 1, **caractérisé en ce que** l'étape consistant à charger le résultat de détection d'état actuel comprend : le fait d'activer un module de communication sans fil, via le défibrillateur, pour transmettre le résultat de détection d'état actuel selon une manière de communication sans fil, et de désactiver le module de communication sans fil une fois que le résultat de détection d'état est transmis.

4. Procédé de gestion de détection d'état selon la revendication 3, comprenant en outre : le fait de continuer à réaliser une connexion sans fil pour un nombre d'instants prédéfini lorsque le défibrillateur détermine que la connexion sans fil a échoué après avoir activé le module de communication sans fil ; et de mémoriser le résultat de détection d'état actuel et d'amener le défibrillateur à entrer dans un état de veille lorsque la connexion sans fil n'est toujours pas établie.

5. Procédé de gestion de détection d'état selon l'une quelconque des revendications 1 à 4, comprenant en outre :
le fait de fournir un terminal ; et
le fait de générer une interface de gestion via le terminal après avoir reçu des informations d'identification de connexion d'utilisateur, l'interface de gestion comprend une zone d'informations de dispositif destinée à afficher des informations de dispositif d'une pluralité de défibrillateurs associés aux informations d'identification de connexion d'utilisateur ;
les informations de dispositif de l'un quelconque des défibrillateurs comprennent au moins un élément parmi un élément d'état de dispositif, un élément de dernier instant de chargement d'un résultat de détection d'état, un élément de numéro de série de dispositif, un élément d'emplacement de dispositif, un élément d'état de maintenance, un élément d'informations d'utilisateur associé, et un élément de rappel de mise à niveau.

6. Procédé de gestion de détection d'état selon la revendication 5, comprenant en outre : le fait de détecter, via le terminal, un clic sur l'interface de gestion ; et de générer une interface de rapport de détection d'état de dispositif lorsque le clic sur l'élément d'état de dispositif de l'un quelconque des défibrillateurs, l'élément de dernier instant de chargement du résultat de détection d'état, ou l'élément de numéro de série de dispositif est détecté par le terminal, l'interface de rapport de détection d'état de dispositif comprend au moins le résultat de détection d'état chargé en dernier par le défibrillateur.

7. Procédé de gestion de détection d'état selon la revendication 6, **caractérisé en ce que** le résultat de détection d'état comprend des résultats de test des défibrillateurs, et, en outre comprenant : le fait d'envoyer, via le terminal, le résultat de détection d'état à un utilisateur associé à l'un des défibrillateurs, lorsque l'un quelconque des résultats de test de l'un des défibrillateurs indique une défaillance ; et/ou, le fait d'envoyer, via le terminal, un message de rappel à l'utilisateur, lorsque le terminal n'a pas reçu le résultat de détection d'état de l'un des défibrillateurs après un laps de temps prédéfini.

8. Procédé de gestion de détection d'état selon la revendication 5, **caractérisé en ce que** l'interface de gestion comprend en outre une zone de gestion de dispositif destinée à permettre à un utilisateur de mettre à niveau un programme du défibrillateur d'une manière sans fil ; et/ou
l'interface de gestion comprend en outre une zone de gestion d'utilisateur destinée à gérer des utilisateurs associés aux informations d'identification de connexion d'utilisateur.

9. Défibrillateur, comprenant :
un module de détection d'état (01) destiné à effectuer automatiquement une détection d'état sur le défibrillateur pour obtenir un résultat de détection d'état actuel ;
un module d'envoi (03) destiné à envoyer le résultat de détection d'état actuel ; et
un module de traitement (5) destiné à comparer le résultat de détection d'état actuel au dernier résultat de détection d'état ; à notifier au module d'envoi (03) de charger le résultat de détection d'état actuel lorsque le résultat de détection d'état actuel est différent du dernier résultat de détection d'état, et à mettre fin à la détection d'état ; à déterminer si de multiples résultats de détection d'état obtenus de multiples instants de détection d'état dans un laps de temps prédéfini sont tous les mêmes lorsque le résultat de détection d'état actuel est le même que le dernier résultat de détection d'état ; et à notifier au module d'envoi (03) de charger le résultat de détection d'état actuel si les multiples résultats de détection d'état sont tous les mêmes, et dans le cas contraire de ne pas charger le résultat de détection d'état actuel, et à mettre fin à la détection d'état.

10. Défibrillateur selon la revendication 9, **caractérisé en ce que** le module de traitement (05) est pourvu d'une pluralité d'instants de détection d'état, et est configuré pour sortir le défibrillateur d'un état de veille à chacun des instants de détection d'état, pour notifier au module de détection d'état (01) d'effectuer la détection d'état sur le défibrillateur, et ensuite pour commander le défibrillateur afin de le faire entrer dans état de veille une fois que la détection d'état est terminée.

11. Défibrillateur selon la revendication 9, **caractérisé en ce que** le module d'envoi (03) comprend un module de communication sans fil (03a), et le module de traitement (05) active le module de communication sans fil (03a) pour transmettre le résultat de détection d'état actuel selon une manière de communication sans fil lorsque le résultat de détection d'état doit être chargé, et désactive le module de communication sans fil une fois que le résultat de détection d'état est transmis.

12. Défibrillateur selon la revendication 11, où après que le module de traitement (05) active le module de communication sans fil (03a), le module de traitement (05) commande le module de communication sans fil (03a) pour continuer à réaliser une connexion sans fil pour un nombre d'instants prédéfini lorsque la connexion sans fil du module de communication sans fil (03a) a échoué, et le module de traitement (05) commande le défibrillateur pour lui faire mémoriser le résultat de détection d'état actuel et le faire entrer dans un état de veille lorsque la connexion sans fil n'est toujours pas établie.

13. Dispositif de gestion destiné à un défibrillateur comprenant une mémoire et un processeur, le processeur exécute les processus suivants en appelant un programme dans la mémoire :
générer une interface de gestion (S212) après avoir reçu des informations d'identification de connexion d'utilisateur ;
afficher (S214) l'interface de gestion, l'interface de gestion comprend au moins une zone d'informations de dispositif destinée à afficher des informations de dispositif d'une pluralité de défibrillateurs associés aux informations d'identification de connexion d'utilisateur, les informations de dispositif comprenant au moins un élément de numéro de série de dispositif et un résultat de détection d'état correspondant au numéro de série de dispositif ;
charger et afficher le résultat de détection d'état en réponse à l'une des situations suivantes :
(1) les multiples résultats de détection d'état obtenus de multiples instants de détections d'état du défibrillateur dans un laps de temps prédéfini sont les mêmes ; et
(2) deux résultats de détection d'état consécutifs du défibrillateur sont différents.

14. Programme informatique comprenant des instructions lesquelles, lorsque le programme est exécuté par un ordinateur, amènent l'ordinateur à commander un défibrillateur selon l'une quelconque des revendications 9 à 12 afin d'exécuter le programme informatique comprenant le procédé de gestion de détection d'état selon les revendications 1 à 8.
